# EUROPEAN PATENT APPLICATION

(11) **EP 3 366 704 A1**
(43) Date of publication of application: **29.08.2018**
(21) Application number: 17158565.6
(22) Date of filing: 28.02.2017
(51) Int. Cl.: C07K 16/28, C07K 16/30, C07K 7/06, C07K 16/40, A61K 39/395, A61K 39/00

(54) **ANTIBODIES SPECIFIC FOR MMP1/HLA-A2 COMPLEX**

(71) Applicant: Affimed GmbH, 69120 Heidelberg (DE)
(72) Inventor: Molkenthin, Vera, 92723 Tännesberg (DE); Fucek, Ivica, 65795 Hattersheim (DE); Ellwanger, Kristina, 69120 Heidelberg (DE); Weichel, Michael, 65474 Bischofsheim (DE); Reusch, Uwe, 67487 Maikammmer (DE); Knackmuss, Stefan, 68723 Planckstadt (DE); Tesar, Michael, 86316 Friedberg (DE); Rajkovic, Erich, 69198 Schrießheim (DE); Treder, Martin, 69120 Heidelberg (DE)
(74) Representative: Schüssler, Andrea

(57) **Abstract**

The invention provides multispecific antigen-binding molecules having a specificity for a complex of matrix metallopeptidase 1 (MMP1) and the human leukocyte antigen A2 (HLA-A2), and a further specificity for engaging an immune effector cell, such as a T-cell via CD3 receptor or a NK-cell via CD16A receptor. The invention further includes such multispecific antigen binding molecules for use in immunotherapy, in particular for the treatment of a MMP1⁺ cancer.

SEQUENCE LISTING

<110>  Affimed GmbH

<120>  Antibodies specific for MMP1/HLA-A2 complex

<130>  A3315EP

<160>  54

<170>  PatentIn version 3.5

<210>  1
<211>  5
<212>  PRT
<213>  artificial sequence

<220>
<223>  CDR

<400>  1

Thr Phe Trp Ile Gly
1               5


<210>  2
<211>  17
<212>  PRT
<213>  artificial sequence

<220>
<223>  CDR

<400>  2

Ser Ile Tyr Pro Gly Asp Ser Asn Thr Ile Tyr Ser Pro Ser Phe Gln
1               5                   10                  15


Gly



<210>  3
<211>  12
<212>  PRT
<213>  artificial sequence

<220>
<223>  CDR

<400>  3

Ile Arg Asp Gly Tyr Ser Tyr Asp Ala Phe Asp Leu
1               5                   10


<210>  4
<211>  14
<212>  PRT
<213>  artificial sequence

<220>
<223>  CDR

<400>  4

Thr Gly Thr Ser Ser Asp Val Gly Gly Tyr Asn Tyr Val Ser
1               5                   10


<210>  5
<211>  7
<212>  PRT
<213>  artificial sequence

<220>
<223>  CDR

<400>  5

Asp Val Ser Asn Arg Pro Ser
1               5


<210>  6
<211>  12
<212>  PRT
<213>  artificial sequence

<220>
<223>  CDR

<400>  6

Ser Ser Tyr Thr Ser Ser Ser Thr Leu Ala Tyr Val
1               5                   10


<210>  7
<211>  5
<212>  PRT
<213>  artificial sequence

<220>
<223>  CDR

<400>  7

Ser Tyr Ala Met His
1               5


<210>  8
<211>  17
<212>  PRT
<213>  artificial sequence

<220>
<223>  CDR

<400>  8

Val Ile Ser Tyr Asp Gly Ser Asn Lys Tyr Tyr Ala Asp Ser Val Lys
1               5                   10                  15

Gly

<210> 9
<211> 11
<212> PRT
<213> artificial sequence

<220>
<223> CDR

<400> 9

Asp Gly Gly Tyr Tyr His Tyr Gly Leu Asp Val
1               5                   10

<210> 10
<211> 11
<212> PRT
<213> artificial sequence

<220>
<223> CDR

<400> 10

Ser Gly Asp Lys Leu Gly Asp Lys Tyr Ala Ser
1               5                   10

<210> 11
<211> 7
<212> PRT
<213> artificial sequence

<220>
<223> CDR

<400> 11

Gln Asp Ala Lys Arg Pro Ser
1               5

<210> 12
<211> 9
<212> PRT
<213> artificial sequence

<220>
<223> CDR

<400> 12

Gln Ala Trp Asp Ser Ser Thr Gly Val
1               5

<210> 13

<211>  5
<212>  PRT
<213>  artificial sequence

<220>
<223>  CDR

<400>  13

Ser Tyr Ala Met His
1               5


<210>  14
<211>  17
<212>  PRT
<213>  artificial sequence

<220>
<223>  CDR

<400>  14

Val Ile Ser Tyr Asp Gly Ser Asn Lys Tyr Tyr Ala Asp Ser Val Lys
1               5                   10                  15


Gly



<210>  15
<211>  13
<212>  PRT
<213>  artificial sequence

<220>
<223>  CDR

<400>  15

Asp His Thr Glu Gly Tyr Tyr Tyr Tyr Gly Met Asp Val
1               5                   10


<210>  16
<211>  11
<212>  PRT
<213>  artificial sequence

<220>
<223>  CDR

<400>  16

Ser Gly Asp Lys Leu Gly Asp Lys Tyr Val Ser
1               5                   10


<210>  17
<211>  7
<212>  PRT
<213>  artificial sequence

<220>
<223>  CDR

<400>  17

Gln Asp Ser Lys Arg Pro Ser
1               5


<210>  18
<211>  9
<212>  PRT
<213>  artificial sequence

<220>
<223>  CDR

<400>  18

Gln Ala Trp Asp Ser Ser Thr Val Val
1               5


<210>  19
<211>  5
<212>  PRT
<213>  artificial sequence

<220>
<223>  CDR

<400>  19

Ser Tyr Gly Met His
1               5


<210>  20
<211>  17
<212>  PRT
<213>  artificial sequence

<220>
<223>  CDR

<400>  20

Val Ile Ser Tyr Asp Gly Ser Asn Lys Tyr Tyr Ala Asp Ser Val Lys
1               5                   10                  15


Gly



<210>  21
<211>  13
<212>  PRT
<213>  artificial sequence

<220>
<223>  CDR

<400>  21

Asp Ser Phe Trp Gly Ser Tyr Tyr Tyr Gly Met Asp Val
1               5                   10


<210>  22
<211>  11
<212>  PRT
<213>  artificial sequence

<220>
<223>  CDR

<400>  22

Ser Gly Asp Lys Leu Gly Asp Arg Tyr Ala Ser
1               5                   10


<210>  23
<211>  7
<212>  PRT
<213>  artificial sequence

<220>
<223>  CDR

<400>  23

Gln Asp Asn Lys Arg Pro Ser
1               5


<210>  24
<211>  10
<212>  PRT
<213>  artificial sequence

<220>
<223>  CDR

<400>  24

Gln Ala Trp Asp Ser Ser Thr Ala Lys Val
1               5                   10


<210>  25
<211>  121
<212>  PRT
<213>  artificial sequence

<220>
<223>  VH

<400>  25

Gln Val Gln Leu Val Gln Ser Gly Ala Glu Ala Lys Lys Pro Gly Glu
1               5                   10                  15

Ser Leu Arg Ile Ser Cys Arg Ala Ser Gly Tyr Met Phe Ser Thr Phe
            20                  25                  30

Trp Ile Gly Trp Val Arg Gln Met Pro Gly Lys Gly Leu Glu Trp Val
        35                  40                  45

Ala Ser Ile Tyr Pro Gly Asp Ser Asn Thr Ile Tyr Ser Pro Ser Phe
    50                  55                  60

Gln Gly Gln Val Thr Ile Ser Ala Asp Lys Ser Ile Asn Thr Thr Tyr
65                  70                  75                  80

Leu Gln Trp Ser Gly Leu Lys Ala Ser Asp Thr Ala Thr Tyr Tyr Cys
                85                  90                  95

Ala Lys Ile Arg Asp Gly Tyr Ser Tyr Asp Ala Phe Asp Leu Trp Gly
            100                 105                 110

Gln Gly Thr Met Val Thr Val Ser Ser
        115                 120

<210>  26
<211>  112
<212>  PRT
<213>  artificial sequence

<220>
<223>  VL

<400>  26

Gln Ser Ala Leu Thr Gln Pro Ala Ser Val Ser Gly Ser Pro Gly Gln
1               5                   10                  15

Ser Ile Thr Ile Ser Cys Thr Gly Thr Ser Ser Asp Val Gly Gly Tyr
            20                  25                  30

Asn Tyr Val Ser Trp Tyr Gln Gln His Pro Gly Lys Ala Pro Lys Leu
        35                  40                  45

Met Ile Tyr Asp Val Ser Asn Arg Pro Ser Gly Val Ser Asn Arg Phe
    50                  55                  60

Ser Gly Ser Lys Ser Gly Asn Thr Ala Ser Leu Thr Ile Ser Gly Leu
65                  70                  75                  80

Gln Ala Glu Asp Glu Ala Asp Tyr Tyr Cys Ser Ser Tyr Thr Ser Ser
                85                  90                  95

Ser Thr Leu Ala Tyr Val Phe Gly Thr Gly Thr Lys Leu Thr Val Leu

            100                 105                 110

<210> 27
<211> 120
<212> PRT
<213> artificial sequence

<220>
<223> VH

<400> 27

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Ala Met His Trp Val Arg Gln Ala Pro Gly Lys Gly Leu Glu Trp Val
        35                  40                  45

Ala Val Ile Ser Tyr Asp Gly Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Thr Glu Asp Thr Ala Val Tyr Tyr Cys
                85                  90                  95

Ala Arg Asp Gly Gly Tyr Tyr His Tyr Gly Leu Asp Val Trp Gly Gln
            100                 105                 110

Gly Thr Thr Val Thr Val Ser Ser
        115                 120

<210> 28
<211> 106
<212> PRT
<213> artificial sequence

<220>
<223> VL

<400> 28

Ser Tyr Glu Leu Thr Gln Pro Pro Ser Val Ser Val Ser Pro Gly Gln
1               5                   10                  15

Thr Ala Ser Ile Thr Cys Ser Gly Asp Lys Leu Gly Asp Lys Tyr Ala
            20                  25                  30

Ser Trp Tyr Gln Arg Lys Pro Gly Gln Ser Pro Val Leu Leu Ile Tyr
        35                  40                  45

Gln Asp Ala Lys Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Met
65                  70                  75                  80

Asp Glu Ala Asp Tyr Tyr Cys Gln Ala Trp Asp Ser Ser Thr Gly Val
                85                  90                  95

Phe Gly Gly Gly Thr Lys Leu Thr Val Leu
            100                 105

<210>  29
<211>  122
<212>  PRT
<213>  artificial sequence

<220>
<223>  VH

<400>  29

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Ala Met His Trp Val Arg Gln Ala Pro Gly Lys Gly Leu Glu Trp Val
        35                  40                  45

Ala Val Ile Ser Tyr Asp Gly Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp His Thr Glu Gly Tyr Tyr Tyr Tyr Gly Met Asp Val Trp
            100                 105                 110

Gly Gln Gly Thr Thr Val Thr Val Ser Ser
        115                 120

<210>  30

<211>  106
<212>  PRT
<213>  artificial sequence

<220>
<223>  VL

<400>  30

Ser Tyr Glu Leu Thr Gln Pro Pro Ser Val Ser Val Ser Pro Gly Gln
1               5                   10                  15

Thr Ala Ser Ile Thr Cys Ser Gly Asp Lys Leu Gly Asp Lys Tyr Val
            20                  25                  30

Ser Trp Tyr Gln Gln Lys Pro Gly Gln Ser Pro Val Leu Val Ile Tyr
        35                  40                  45

Gln Asp Ser Lys Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Met
65                  70                  75                  80

Asp Glu Ala Asp Tyr Tyr Cys Gln Ala Trp Asp Ser Ser Thr Val Val
                85                  90                  95

Phe Gly Gly Gly Thr Lys Val Thr Val Leu
            100                 105


<210>  31
<211>  122
<212>  PRT
<213>  artificial sequence

<220>
<223>  VH

<400>  31

Gln Val Gln Leu Leu Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Ile Ser Tyr Asp Gly Ser Asn Lys Tyr Tyr Ala Asp Ser Val
    50                  55                  60

Lys Gly Arg Phe Thr Ile Ser Arg Asp Asp Ala Lys Asn Thr Leu Tyr
65                  70                  75                  80

Leu Gln Met Asn Ser Leu Arg Ala Glu Asp Thr Ala Val Tyr Tyr Cys
                85                  90                  95

Val Arg Asp Ser Phe Trp Gly Ser Tyr Tyr Tyr Gly Met Asp Val Trp
            100                 105                 110

Gly His Gly Thr Thr Val Thr Val Ser Ser
        115                 120

<210>  32
<211>  107
<212>  PRT
<213>  artificial sequence

<220>
<223>  VL

<400>  32

Ser Tyr Glu Leu Thr Gln Pro Pro Ser Val Ser Val Ser Pro Gly Gln
1               5                   10                  15

Thr Ala Thr Ile Thr Cys Ser Gly Asp Lys Leu Gly Asp Arg Tyr Ala
            20                  25                  30

Ser Trp Tyr Glu Gln Arg Pro Gly Gln Ser Pro Val Leu Val Ile Tyr
        35                  40                  45

Gln Asp Asn Lys Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Met
65                  70                  75                  80

Asp Glu Ala Asp Tyr Tyr Cys Gln Ala Trp Asp Ser Ser Thr Ala Lys
                85                  90                  95

Val Phe Gly Thr Gly Thr Lys Leu Thr Val Leu
            100                 105

<210>  33
<211>  125
<212>  PRT
<213>  artificial sequence

<220>
<223>  VH CD3

<400>  33

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Thr Tyr
            20                  25                  30

Ala Met Asn Trp Val Arg Gln Ala Pro Gly Lys Gly Leu Glu Trp Val
        35                  40                  45

Gly Arg Ile Arg Ser Lys Tyr Asn Asn Tyr Ala Thr Tyr Tyr Ala Asp
    50                  55                  60

Ser Val Lys Asp Arg Phe Thr Ile Ser Arg Asp Asp Ser Lys Asn Ser
65                  70                  75                  80

Leu Tyr Leu Gln Met Asn Ser Leu Lys Thr Glu Asp Thr Ala Val Tyr
                85                  90                  95

Tyr Cys Ala Arg His Gly Asn Phe Gly Asn Ser Tyr Val Ser Tyr Phe
            100                 105                 110

Ala Tyr Trp Gly Gln Gly Thr Leu Val Thr Val Ser Ser
        115                 120                 125

<210>  34
<211>  110
<212>  PRT
<213>  artificial sequence

<220>
<223>  VL

<400>  34

Asp Ile Gln Met Thr Gln Ser Pro Ser Ser Leu Ser Ala Ser Val Gly
1               5                   10                  15

Asp Arg Val Thr Ile Thr Cys Arg Ser Ser Thr Gly Ala Val Thr Thr
            20                  25                  30

Ser Asn Tyr Ala Asn Trp Val Gln Gln Lys Pro Gly Lys Ala Pro Lys
        35                  40                  45

Ala Leu Ile Gly Gly Thr Asn Lys Arg Ala Pro Gly Val Pro Ser Arg
    50                  55                  60

Phe Ser Gly Ser Leu Ile Gly Asp Lys Ala Thr Leu Thr Ile Ser Ser
65                  70                  75                  80

Leu Gln Pro Glu Asp Phe Ala Thr Tyr Tyr Cys Ala Leu Trp Tyr Ser
                85                  90                  95

Asn Leu Trp Val Phe Gly Gln Gly Thr Lys Val Glu Ile Lys
            100                 105                 110

<210>  35
<211>  120
<212>  PRT
<213>  artificial sequence

<220>
<223>  VH CD16A

<400>  35

Gln Val Gln Leu Val Gln Ser Gly Ala Glu Val Lys Lys Pro Gly Glu
1               5                   10                  15

Ser Leu Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Ile Ile Asn Pro Ser Gly Gly Ser Thr Ser Tyr Ala Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Gly Ser Ala Tyr Tyr Tyr Asp Phe Ala Asp Tyr Trp Gly Gln
            100                 105                 110

Gly Thr Leu Val Thr Val Ser Ser
        115                 120

<210>  36
<211>  106
<212>  PRT
<213>  artificial sequence

<220>
<223>  VL CD16A

<400>  36

Ser Tyr Val Leu Thr Gln Pro Ser Ser Val Ser Val Ala Pro Gly Gln
1               5                   10                  15

Thr Ala Thr Ile Ser Cys Gly Gly His Asn Ile Gly Ser Lys Asn Val
            20                  25                  30

His Trp Tyr Gln Gln Arg Pro Gly Gln Ser Pro Val Leu Val Ile Tyr
        35                  40                  45

Gln Asp Asn Lys Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Met
65                  70                  75                  80

Asp Glu Ala Asp Tyr Tyr Cys Gln Val Trp Asp Asn Tyr Ser Val Leu
                85                  90                  95

Phe Gly Gly Gly Thr Lys Leu Thr Val Leu
            100                 105

<210>  37
<211>  274
<212>  PRT
<213>  artificial sequence

<220>
<223>  scFv

<400>  37

Gln Val Gln Leu Val Gln Ser Gly Ala Glu Ala Lys Lys Pro Gly Glu
1               5                   10                  15

Ser Leu Arg Ile Ser Cys Arg Ala Ser Gly Tyr Met Phe Ser Thr Phe
            20                  25                  30

Trp Ile Gly Trp Val Arg Gln Met Pro Gly Lys Gly Leu Glu Trp Val
        35                  40                  45

Ala Ser Ile Tyr Pro Gly Asp Ser Asn Thr Ile Tyr Ser Pro Ser Phe
    50                  55                  60

Gln Gly Gln Val Thr Ile Ser Ala Asp Lys Ser Ile Asn Thr Thr Tyr
65                  70                  75                  80

Leu Gln Trp Ser Gly Leu Lys Ala Ser Asp Thr Ala Thr Tyr Tyr Cys
                85                  90                  95

Ala Lys Ile Arg Asp Gly Tyr Ser Tyr Asp Ala Phe Asp Leu Trp Gly
            100                 105                 110

Gln Gly Thr Met Val Thr Val Ser Ser Gly Gly Ser Gly Gly Ser Gly
        115                 120                 125

Gly Ser Gly Gly Ser Gly Gly Ser Gly Gly Ser Gln Ser Ala Leu Thr
    130                 135                 140

Gln Pro Ala Ser Val Ser Gly Ser Pro Gly Gln Ser Ile Thr Ile Ser
145                 150                 155                 160

Cys Thr Gly Thr Ser Ser Asp Val Gly Gly Tyr Asn Tyr Val Ser Trp
                165                 170                 175

Tyr Gln Gln His Pro Gly Lys Ala Pro Lys Leu Met Ile Tyr Asp Val
            180                 185                 190

Ser Asn Arg Pro Ser Gly Val Ser Asn Arg Phe Ser Gly Ser Lys Ser
        195                 200                 205

Gly Asn Thr Ala Ser Leu Thr Ile Ser Gly Leu Gln Ala Glu Asp Glu
    210                 215                 220

Ala Asp Tyr Tyr Cys Ser Ser Tyr Thr Ser Ser Ser Thr Leu Ala Tyr
225                 230                 235                 240

Val Phe Gly Thr Gly Thr Lys Leu Thr Val Leu Ala Ala Ala Gly Ser
                245                 250                 255

Glu Gln Lys Leu Ile Ser Glu Glu Asp Leu Asn Ser His His His His
            260                 265                 270

His His

<210>  38
<211>  267
<212>  PRT
<213>  artificial sequence

<220>
<223>  scFv

<400>  38

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Ala Met His Trp Val Arg Gln Ala Pro Gly Lys Gly Leu Glu Trp Val

35                  40                  45

Ala Val Ile Ser Tyr Asp Gly Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Thr Glu Asp Thr Ala Val Tyr Tyr Cys
                85                  90                  95

Ala Arg Asp Gly Gly Tyr Tyr His Tyr Gly Leu Asp Val Trp Gly Gln
            100                 105                 110

Gly Thr Thr Val Thr Val Ser Ser Gly Gly Ser Gly Gly Ser Gly Gly
        115                 120                 125

Ser Gly Gly Ser Gly Gly Ser Gly Gly Ser Ser Tyr Glu Leu Thr Gln
    130                 135                 140

Pro Pro Ser Val Ser Val Ser Pro Gly Gln Thr Ala Ser Ile Thr Cys
145                 150                 155                 160

Ser Gly Asp Lys Leu Gly Asp Lys Tyr Ala Ser Trp Tyr Gln Arg Lys
                165                 170                 175

Pro Gly Gln Ser Pro Val Leu Leu Ile Tyr Gln Asp Ala Lys Arg Pro
            180                 185                 190

Ser Gly Ile Pro Glu Arg Phe Ser Gly Ser Asn Ser Gly Asn Thr Ala
        195                 200                 205

Thr Leu Thr Ile Ser Gly Thr Gln Ala Met Asp Glu Ala Asp Tyr Tyr
    210                 215                 220

Cys Gln Ala Trp Asp Ser Ser Thr Gly Val Phe Gly Gly Gly Thr Lys
225                 230                 235                 240

Leu Thr Val Leu Ala Ala Ala Gly Ser Glu Gln Lys Leu Ile Ser Glu
                245                 250                 255

Glu Asp Leu Asn Ser His His His His His His
            260                 265

<210>  39
<211>  273
<212>  PRT
<213>  artificial sequence

<220>
<223>  scFv

<400>  39

Gln Val Gln Leu Leu Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Ile Ser Tyr Asp Gly Ser Asn Lys Tyr Tyr Ala Asp Ser Val
    50                  55                  60

Lys Gly Arg Phe Thr Ile Ser Arg Asp Asp Ala Lys Asn Thr Leu Tyr
65                  70                  75                  80

Leu Gln Met Asn Ser Leu Arg Ala Glu Asp Thr Ala Val Tyr Tyr Cys
                85                  90                  95

Val Arg Asp Ser Phe Trp Gly Ser Tyr Tyr Tyr Gly Met Asp Val Trp
            100                 105                 110

Gly His Gly Thr Thr Val Thr Val Ser Ser Leu Gly Gly Ser Gly Gly
        115                 120                 125

Ser Gly Gly Ser Gly Gly Ser Gly Gly Ser Gly Gly Ser Asn Ala Ser
    130                 135                 140

Tyr Glu Leu Thr Gln Pro Pro Ser Val Ser Val Ser Pro Gly Gln Thr
145                 150                 155                 160

Ala Thr Ile Thr Cys Ser Gly Asp Lys Leu Gly Asp Arg Tyr Ala Ser
                165                 170                 175

Trp Tyr Glu Gln Arg Pro Gly Gln Ser Pro Val Leu Val Ile Tyr Gln
            180                 185                 190

Asp Asn Lys Arg Pro Ser Gly Ile Pro Glu Arg Phe Ser Gly Ser Asn
        195                 200                 205

Ser Gly Asn Thr Ala Thr Leu Thr Ile Ser Gly Thr Gln Ala Met Asp
    210                 215                 220

Glu Ala Asp Tyr Tyr Cys Gln Ala Trp Asp Ser Ser Thr Ala Lys Val

225                 230                 235                 240

Phe Gly Thr Gly Thr Lys Leu Thr Val Leu Ala Ala Ala Gly Ser Glu
                245                 250                 255

Gln Lys Leu Ile Ser Glu Glu Asp Leu Asn Ser His His His His His
            260                 265                 270

His

<210>  40
<211>  272
<212>  PRT
<213>  artificial sequence

<220>
<223>  scFv

<400>  40

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Ala Met His Trp Val Arg Gln Ala Pro Gly Lys Gly Leu Glu Trp Val
        35                  40                  45

Ala Val Ile Ser Tyr Asp Gly Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp His Thr Glu Gly Tyr Tyr Tyr Tyr Gly Met Asp Val Trp
            100                 105                 110

Gly Gln Gly Thr Thr Val Thr Val Ser Ser Leu Gly Gly Ser Gly Gly
        115                 120                 125

Ser Gly Gly Ser Gly Gly Ser Gly Gly Ser Gly Gly Ser Asn Ala Ser
    130                 135                 140

Tyr Glu Leu Thr Gln Pro Pro Ser Val Ser Val Ser Pro Gly Gln Thr
145                 150                 155                 160

Ala Ser Ile Thr Cys Ser Gly Asp Lys Leu Gly Asp Lys Tyr Val Ser
                165                 170                 175

Trp Tyr Gln Gln Lys Pro Gly Gln Ser Pro Val Leu Val Ile Tyr Gln
            180                 185                 190

Asp Ser Lys Arg Pro Ser Gly Ile Pro Glu Arg Phe Ser Gly Ser Asn
        195                 200                 205

Ser Gly Asn Thr Ala Thr Leu Thr Ile Ser Gly Thr Gln Ala Met Asp
    210                 215                 220

Glu Ala Asp Tyr Tyr Cys Gln Ala Trp Asp Ser Ser Thr Val Val Phe
225                 230                 235                 240

Gly Gly Gly Thr Lys Val Thr Val Leu Ala Ala Ala Gly Ser Glu Gln
                245                 250                 255

Lys Leu Ile Ser Glu Glu Asp Leu Asn Ser His His His His His His
            260                 265                 270

<210>  41
<211>  4
<212>  PRT
<213>  artificial sequence

<220>
<223>  linker

<400>  41

Gly Gly Ser Gly
1

<210>  42
<211>  6
<212>  PRT
<213>  artificial sequence

<220>
<223>  linker

<400>  42

Gly Gly Ser Gly Gly Ser
1               5

<210>  43
<211>  5
<212>  PRT
<213>  artificial sequence

<220>
<223>  linker

<400>  43

Gly Gly Ser Gly Gly
1               5


<210>  44
<211>  9
<212>  PRT
<213>  artificial sequence

<220>
<223>  linker

<400>  44

Gly Gly Ser Gly Gly Ser Gly Gly Ser
1               5


<210>  45
<211>  9
<212>  PRT
<213>  homo sapiens

<400>  45

Tyr Thr Phe Ser Gly Asp Val Gln Leu
1               5


<210>  46
<211>  120
<212>  PRT
<213>  artificial sequence

<220>
<223>  VH CD16A

<400>  46

Gln Val Gln Leu Val Gln Ser Gly Ala Glu Val Lys Lys Pro Gly Glu
1               5                   10                  15


Ser Leu Lys Val Ser Cys Lys Ala Ser Gly Tyr Thr Phe Thr Ser Tyr
            20                  25                  30


Tyr Met His Trp Val Arg Gln Ala Pro Gly Gln Gly Leu Glu Trp Met
        35                  40                  45


Gly Ala Ile Glu Pro Met Tyr Gly Ser Thr Ser Tyr Ala Gln Lys Phe
    50                  55                  60


Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Gly Ser Ala Tyr Tyr Tyr Asp Phe Ala Asp Tyr Trp Gly Gln
            100                 105                 110

Gly Thr Leu Val Thr Val Ser Ser
        115                 120

<210>  47
<211>  106
<212>  PRT
<213>  artificial sequence

<220>
<223>  VL CD16A

<400>  47

Ser Tyr Val Leu Thr Gln Pro Ser Ser Val Ser Val Ala Pro Gly Gln
1               5                   10                  15

Thr Ala Thr Ile Ser Cys Gly Gly His Asn Ile Gly Ser Lys Asn Val
            20                  25                  30

His Trp Tyr Gln Gln Arg Pro Gly Gln Ser Pro Val Leu Val Ile Tyr
        35                  40                  45

Gln Asp Asn Lys Arg Pro Ser Gly Ile Pro Glu Arg Phe Ser Gly Ser
    50                  55                  60

Asn Ser Gly Asn Thr Ala Thr Leu Thr Ile Ser Gly Thr Gln Ala Met
65                  70                  75                  80

Asp Glu Ala Asp Tyr Tyr Cys Gln Val Trp Asp Asn Tyr Ser Val Leu
                85                  90                  95

Phe Gly Gly Gly Thr Lys Leu Thr Val Leu
            100                 105

<210>  48
<211>  495
<212>  PRT
<213>  artificial sequence

<220>
<223>  tandem diabody

<400>  48

Asp Ile Gln Met Thr Gln Ser Pro Ser Ser Leu Ser Ala Ser Val Gly
1               5                   10                  15

Asp Arg Val Thr Ile Thr Cys Arg Ser Ser Thr Gly Ala Val Thr Thr
            20                  25                  30

Ser Asn Tyr Ala Asn Trp Val Gln Gln Lys Pro Gly Lys Ala Pro Lys
        35                  40                  45

Ala Leu Ile Gly Gly Thr Asn Lys Arg Ala Pro Gly Val Pro Ser Arg
    50                  55                  60

Phe Ser Gly Ser Leu Ile Gly Asp Lys Ala Thr Leu Thr Ile Ser Ser
65                  70                  75                  80

Leu Gln Pro Glu Asp Phe Ala Thr Tyr Tyr Cys Ala Leu Trp Tyr Ser
                85                  90                  95

Asn Leu Trp Val Phe Gly Gln Gly Thr Lys Val Glu Ile Lys Gly Gly
            100                 105                 110

Ser Gly Gly Ser Gln Val Gln Leu Val Gln Ser Gly Ala Glu Ala Lys
        115                 120                 125

Lys Pro Gly Glu Ser Leu Arg Ile Ser Cys Arg Ala Ser Gly Tyr Met
    130                 135                 140

Phe Ser Thr Phe Trp Ile Gly Trp Val Arg Gln Met Pro Gly Lys Gly
145                 150                 155                 160

Leu Glu Trp Val Ala Ser Ile Tyr Pro Gly Asp Ser Asn Thr Ile Tyr
                165                 170                 175

Ser Pro Ser Phe Gln Gly Gln Val Thr Ile Ser Ala Asp Lys Ser Ile
            180                 185                 190

Asn Thr Thr Tyr Leu Gln Trp Ser Gly Leu Lys Ala Ser Asp Thr Ala
        195                 200                 205

Thr Tyr Tyr Cys Ala Lys Ile Arg Asp Gly Tyr Ser Tyr Asp Ala Phe
    210                 215                 220

Asp Leu Trp Gly Gln Gly Thr Met Val Thr Val Ser Ser Gly Gly Ser
225                 230                 235                 240

Gly Gln Ser Ala Leu Thr Gln Pro Ala Ser Val Ser Gly Ser Pro Gly
                245                 250                 255

Gln Ser Ile Thr Ile Ser Cys Thr Gly Thr Ser Ser Asp Val Gly Gly

260                 265                 270

Tyr Asn Tyr Val Ser Trp Tyr Gln Gln His Pro Gly Lys Ala Pro Lys
        275                 280                 285

Leu Met Ile Tyr Asp Val Ser Asn Arg Pro Ser Gly Val Ser Asn Arg
    290                 295                 300

Phe Ser Gly Ser Lys Ser Gly Asn Thr Ala Ser Leu Thr Ile Ser Gly
305                 310                 315                 320

Leu Gln Ala Glu Asp Glu Ala Asp Tyr Tyr Cys Ser Ser Tyr Thr Ser
                325                 330                 335

Ser Ser Thr Leu Ala Tyr Val Phe Gly Thr Gly Thr Lys Leu Thr Val
            340                 345                 350

Leu Gly Gly Ser Gly Gly Ser Glu Val Gln Leu Val Glu Ser Gly Gly
        355                 360                 365

Gly Leu Val Gln Pro Gly Gly Ser Leu Arg Leu Ser Cys Ala Ala Ser
    370                 375                 380

Gly Phe Thr Phe Ser Thr Tyr Ala Met Asn Trp Val Arg Gln Ala Pro
385                 390                 395                 400

Gly Lys Gly Leu Glu Trp Val Gly Arg Ile Arg Ser Lys Tyr Asn Asn
                405                 410                 415

Tyr Ala Thr Tyr Tyr Ala Asp Ser Val Lys Asp Arg Phe Thr Ile Ser
            420                 425                 430

Arg Asp Asp Ser Lys Asn Ser Leu Tyr Leu Gln Met Asn Ser Leu Lys
        435                 440                 445

Thr Glu Asp Thr Ala Val Tyr Tyr Cys Ala Arg His Gly Asn Phe Gly
    450                 455                 460

Asn Ser Tyr Val Ser Tyr Phe Ala Tyr Trp Gly Gln Gly Thr Leu Val
465                 470                 475                 480

Thr Val Ser Ser Ala Ala Ala Gly Ser His His His His His His
                485                 490                 495

<210>  49
<211>  488
<212>  PRT
<213>  artificial sequence

<220>
<223>  tandem diabody

<400>  49

Asp Ile Gln Met Thr Gln Ser Pro Ser Ser Leu Ser Ala Ser Val Gly
1               5                   10                  15

Asp Arg Val Thr Ile Thr Cys Arg Ser Ser Thr Gly Ala Val Thr Thr
            20                  25                  30

Ser Asn Tyr Ala Asn Trp Val Gln Gln Lys Pro Gly Lys Ala Pro Lys
        35                  40                  45

Ala Leu Ile Gly Gly Thr Asn Lys Arg Ala Pro Gly Val Pro Ser Arg
    50                  55                  60

Phe Ser Gly Ser Leu Ile Gly Asp Lys Ala Thr Leu Thr Ile Ser Ser
65                  70                  75                  80

Leu Gln Pro Glu Asp Phe Ala Thr Tyr Tyr Cys Ala Leu Trp Tyr Ser
                85                  90                  95

Asn Leu Trp Val Phe Gly Gln Gly Thr Lys Val Glu Ile Lys Gly Gly
            100                 105                 110

Ser Gly Gly Ser Glu Val Gln Leu Val Glu Ser Gly Gly Gly Val Val
        115                 120                 125

Gln Pro Gly Arg Ser Leu Arg Leu Ser Cys Ala Ala Ser Gly Phe Thr
    130                 135                 140

Phe Ser Ser Tyr Ala Met His Trp Val Arg Gln Ala Pro Gly Lys Gly
145                 150                 155                 160

Leu Glu Trp Val Ala Val Ile Ser Tyr Asp Gly Ser Asn Lys Tyr Tyr
                165                 170                 175

Ala Asp Ser Val Lys Gly Arg Phe Thr Ile Ser Arg Asp Asn Ser Lys
            180                 185                 190

Asn Thr Leu Tyr Leu Gln Met Asn Ser Leu Arg Thr Glu Asp Thr Ala
        195                 200                 205

Val Tyr Tyr Cys Ala Arg Asp Gly Gly Tyr Tyr His Tyr Gly Leu Asp
    210                 215                 220

Val Trp Gly Gln Gly Thr Thr Val Thr Val Ser Ser Gly Gly Ser Gly

225                 230                 235                 240

Ser Tyr Glu Leu Thr Gln Pro Pro Ser Val Ser Val Ser Pro Gly Gln
                245                 250                 255

Thr Ala Ser Ile Thr Cys Ser Gly Asp Lys Leu Gly Asp Lys Tyr Ala
            260                 265                 270

Ser Trp Tyr Gln Arg Lys Pro Gly Gln Ser Pro Val Leu Leu Ile Tyr
        275                 280                 285

Gln Asp Ala Lys Arg Pro Ser Gly Ile Pro Glu Arg Phe Ser Gly Ser
    290                 295                 300

Asn Ser Gly Asn Thr Ala Thr Leu Thr Ile Ser Gly Thr Gln Ala Met
305                 310                 315                 320

Asp Glu Ala Asp Tyr Tyr Cys Gln Ala Trp Asp Ser Ser Thr Gly Val
                325                 330                 335

Phe Gly Gly Gly Thr Lys Leu Thr Val Leu Gly Gly Ser Gly Gly Ser
            340                 345                 350

Glu Val Gln Leu Val Glu Ser Gly Gly Gly Leu Val Gln Pro Gly Gly
        355                 360                 365

Ser Leu Arg Leu Ser Cys Ala Ala Ser Gly Phe Thr Phe Ser Thr Tyr
    370                 375                 380

Ala Met Asn Trp Val Arg Gln Ala Pro Gly Lys Gly Leu Glu Trp Val
385                 390                 395                 400

Gly Arg Ile Arg Ser Lys Tyr Asn Asn Tyr Ala Thr Tyr Tyr Ala Asp
                405                 410                 415

Ser Val Lys Asp Arg Phe Thr Ile Ser Arg Asp Asp Ser Lys Asn Ser
            420                 425                 430

Leu Tyr Leu Gln Met Asn Ser Leu Lys Thr Glu Asp Thr Ala Val Tyr
        435                 440                 445

Tyr Cys Ala Arg His Gly Asn Phe Gly Asn Ser Tyr Val Ser Tyr Phe
    450                 455                 460

Ala Tyr Trp Gly Gln Gly Thr Leu Val Thr Val Ser Ser Ala Ala Ala
465                 470                 475                 480

Gly Ser His His His His His His
                485

<210>  50
<211>  487
<212>  PRT
<213>  artificial sequence

<220>
<223>  tandem diabody

<400>  50

Gln Val Gln Leu Val Gln Ser Gly Ala Glu Val Lys Lys Pro Gly Glu
1               5                   10                  15

Ser Leu Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Ala Ile Glu Pro Met Tyr Gly Ser Thr Ser Tyr Ala Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Gly Ser Ala Tyr Tyr Tyr Asp Phe Ala Asp Tyr Trp Gly Gln
            100                 105                 110

Gly Thr Leu Val Thr Val Ser Ser Gly Gly Ser Gly Gly Ser Gly Gly
        115                 120                 125

Ser Ser Tyr Glu Leu Thr Gln Pro Pro Ser Val Ser Val Ser Pro Gly
    130                 135                 140

Gln Thr Ala Ser Ile Thr Cys Ser Gly Asp Lys Leu Gly Asp Lys Tyr
145                 150                 155                 160

Ala Ser Trp Tyr Gln Arg Lys Pro Gly Gln Ser Pro Val Leu Leu Ile
                165                 170                 175

Tyr Gln Asp Ala Lys Arg Pro Ser Gly Ile Pro Glu Arg Phe Ser Gly
            180                 185                 190

Ser Asn Ser Gly Asn Thr Ala Thr Leu Thr Ile Ser Gly Thr Gln Ala

195                 200                 205

Met Asp Glu Ala Asp Tyr Tyr Cys Gln Ala Trp Asp Ser Ser Thr Gly
    210                 215                 220

Val Phe Gly Gly Gly Thr Lys Leu Thr Val Leu Gly Gly Ser Gly Gly
225                 230                 235                 240

Ser Glu Val Gln Leu Val Glu Ser Gly Gly Gly Val Val Gln Pro Gly
                245                 250                 255

Arg Ser Leu Arg Leu Ser Cys Ala Ala Ser Gly Phe Thr Phe Ser Ser
            260                 265                 270

Tyr Ala Met His Trp Val Arg Gln Ala Pro Gly Lys Gly Leu Glu Trp
        275                 280                 285

Val Ala Val Ile Ser Tyr Asp Gly Ser Asn Lys Tyr Tyr Ala Asp Ser
    290                 295                 300

Val Lys Gly Arg Phe Thr Ile Ser Arg Asp Asn Ser Lys Asn Thr Leu
305                 310                 315                 320

Tyr Leu Gln Met Asn Ser Leu Arg Thr Glu Asp Thr Ala Val Tyr Tyr
                325                 330                 335

Cys Ala Arg Asp Gly Gly Tyr Tyr His Tyr Gly Leu Asp Val Trp Gly
            340                 345                 350

Gln Gly Thr Thr Val Thr Val Ser Ser Gly Gly Ser Gly Gly Ser Gly
        355                 360                 365

Gly Ser Ser Tyr Val Leu Thr Gln Pro Ser Ser Val Ser Val Ala Pro
    370                 375                 380

Gly Gln Thr Ala Thr Ile Ser Cys Gly Gly His Asn Ile Gly Ser Lys
385                 390                 395                 400

Asn Val His Trp Tyr Gln Gln Arg Pro Gly Gln Ser Pro Val Leu Val
                405                 410                 415

Ile Tyr Gln Asp Asn Lys Arg Pro Ser Gly Ile Pro Glu Arg Phe Ser
            420                 425                 430

Gly Ser Asn Ser Gly Asn Thr Ala Thr Leu Thr Ile Ser Gly Thr Gln
        435                 440                 445

Ala Met Asp Glu Ala Asp Tyr Tyr Cys Gln Val Trp Asp Asn Tyr Ser
    450                 455                 460

Val Leu Phe Gly Gly Gly Thr Lys Leu Thr Val Leu Ala Ala Ala Gly
465                 470                 475                 480

Ser His His His His His His
                485

<210>  51
<211>  494
<212>  PRT
<213>  artificial sequence

<220>
<223>  tandem diabody

<400>  51

Gln Val Gln Leu Val Gln Ser Gly Ala Glu Val Lys Lys Pro Gly Glu
1               5                   10                  15

Ser Leu Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Ala Ile Glu Pro Met Tyr Gly Ser Thr Ser Tyr Ala Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Gly Ser Ala Tyr Tyr Tyr Asp Phe Ala Asp Tyr Trp Gly Gln
            100                 105                 110

Gly Thr Leu Val Thr Val Ser Ser Gly Gly Ser Gly Gly Ser Gly Gly
        115                 120                 125

Ser Gln Ser Ala Leu Thr Gln Pro Ala Ser Val Ser Gly Ser Pro Gly
    130                 135                 140

Gln Ser Ile Thr Ile Ser Cys Thr Gly Thr Ser Ser Asp Val Gly Gly
145                 150                 155                 160

Tyr Asn Tyr Val Ser Trp Tyr Gln Gln His Pro Gly Lys Ala Pro Lys

165                 170                 175

Leu Met Ile Tyr Asp Val Ser Asn Arg Pro Ser Gly Val Ser Asn Arg
        180                 185                 190

Phe Ser Gly Ser Lys Ser Gly Asn Thr Ala Ser Leu Thr Ile Ser Gly
    195                 200                 205

Leu Gln Ala Glu Asp Glu Ala Asp Tyr Tyr Cys Ser Ser Tyr Thr Ser
    210                 215                 220

Ser Ser Thr Leu Ala Tyr Val Phe Gly Thr Gly Thr Lys Leu Thr Val
225                 230                 235                 240

Leu Gly Gly Ser Gly Gly Ser Gln Val Gln Leu Val Gln Ser Gly Ala
                245                 250                 255

Glu Ala Lys Lys Pro Gly Glu Ser Leu Arg Ile Ser Cys Arg Ala Ser
        260                 265                 270

Gly Tyr Met Phe Ser Thr Phe Trp Ile Gly Trp Val Arg Gln Met Pro
    275                 280                 285

Gly Lys Gly Leu Glu Trp Val Ala Ser Ile Tyr Pro Gly Asp Ser Asn
    290                 295                 300

Thr Ile Tyr Ser Pro Ser Phe Gln Gly Gln Val Thr Ile Ser Ala Asp
305                 310                 315                 320

Lys Ser Ile Asn Thr Thr Tyr Leu Gln Trp Ser Gly Leu Lys Ala Ser
                325                 330                 335

Asp Thr Ala Thr Tyr Tyr Cys Ala Lys Ile Arg Asp Gly Tyr Ser Tyr
        340                 345                 350

Asp Ala Phe Asp Leu Trp Gly Gln Gly Thr Met Val Thr Val Ser Ser
    355                 360                 365

Gly Gly Ser Gly Gly Ser Gly Gly Ser Ser Tyr Val Leu Thr Gln Pro
    370                 375                 380

Ser Ser Val Ser Val Ala Pro Gly Gln Thr Ala Thr Ile Ser Cys Gly
385                 390                 395                 400

Gly His Asn Ile Gly Ser Lys Asn Val His Trp Tyr Gln Gln Arg Pro
                405                 410                 415

Gly Gln Ser Pro Val Leu Val Ile Tyr Gln Asp Asn Lys Arg Pro Ser
        420                 425                 430

Gly Ile Pro Glu Arg Phe Ser Gly Ser Asn Ser Gly Asn Thr Ala Thr
    435                 440                 445

Leu Thr Ile Ser Gly Thr Gln Ala Met Asp Glu Ala Asp Tyr Tyr Cys
    450                 455                 460

Gln Val Trp Asp Asn Tyr Ser Val Leu Phe Gly Gly Gly Thr Lys Leu
465                 470                 475                 480

Thr Val Leu Ala Ala Ala Gly Ser His His His His His His
                485                 490

<210>  52
<211>  487
<212>  PRT
<213>  artificial sequence

<220>
<223>  tandem diabody

<400>  52

Glu Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Ala Met His Trp Val Arg Gln Ala Pro Gly Lys Gly Leu Glu Trp Val
        35                  40                  45

Ala Val Ile Ser Tyr Asp Gly Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Thr Glu Asp Thr Ala Val Tyr Tyr Cys
                85                  90                  95

Ala Arg Asp Gly Gly Tyr Tyr His Tyr Gly Leu Asp Val Trp Gly Gln
            100                 105                 110

Gly Thr Thr Val Thr Val Ser Ser Gly Gly Ser Gly Gly Ser Gly Gly
        115                 120                 125

Ser Ser Tyr Val Leu Thr Gln Pro Ser Ser Val Ser Val Ala Pro Gly

130                 135                 140

Gln Thr Ala Thr Ile Ser Cys Gly Gly His Asn Ile Gly Ser Lys Asn
145                 150                 155                 160

Val His Trp Tyr Gln Gln Arg Pro Gly Gln Ser Pro Val Leu Val Ile
                165                 170                 175

Tyr Gln Asp Asn Lys Arg Pro Ser Gly Ile Pro Glu Arg Phe Ser Gly
            180                 185                 190

Ser Asn Ser Gly Asn Thr Ala Thr Leu Thr Ile Ser Gly Thr Gln Ala
        195                 200                 205

Met Asp Glu Ala Asp Tyr Tyr Cys Gln Val Trp Asp Asn Tyr Ser Val
    210                 215                 220

Leu Phe Gly Gly Gly Thr Lys Leu Thr Val Leu Gly Gly Ser Gly Gly
225                 230                 235                 240

Ser Gln Val Gln Leu Val Gln Ser Gly Ala Glu Val Lys Lys Pro Gly
                245                 250                 255

Glu Ser Leu Lys Val Ser Cys Lys Ala Ser Gly Tyr Thr Phe Thr Ser
            260                 265                 270

Tyr Tyr Met His Trp Val Arg Gln Ala Pro Gly Gln Gly Leu Glu Trp
        275                 280                 285

Met Gly Ala Ile Glu Pro Met Tyr Gly Ser Thr Ser Tyr Ala Gln Lys
    290                 295                 300

Phe Gln Gly Arg Val Thr Met Thr Arg Asp Thr Ser Thr Ser Thr Val
305                 310                 315                 320

Tyr Met Glu Leu Ser Ser Leu Arg Ser Glu Asp Thr Ala Val Tyr Tyr
                325                 330                 335

Cys Ala Arg Gly Ser Ala Tyr Tyr Tyr Asp Phe Ala Asp Tyr Trp Gly
            340                 345                 350

Gln Gly Thr Leu Val Thr Val Ser Ser Gly Gly Ser Gly Gly Ser Gly
        355                 360                 365

Gly Ser Ser Tyr Glu Leu Thr Gln Pro Pro Ser Val Ser Val Ser Pro
    370                 375                 380

Gly Gln Thr Ala Ser Ile Thr Cys Ser Gly Asp Lys Leu Gly Asp Lys
385                 390                 395                 400

Tyr Ala Ser Trp Tyr Gln Arg Lys Pro Gly Gln Ser Pro Val Leu Leu
                405                 410                 415

Ile Tyr Gln Asp Ala Lys Arg Pro Ser Gly Ile Pro Glu Arg Phe Ser
            420                 425                 430

Gly Ser Asn Ser Gly Asn Thr Ala Thr Leu Thr Ile Ser Gly Thr Gln
        435                 440                 445

Ala Met Asp Glu Ala Asp Tyr Tyr Cys Gln Ala Trp Asp Ser Ser Thr
    450                 455                 460

Gly Val Phe Gly Gly Gly Thr Lys Leu Thr Val Leu Ala Ala Ala Gly
465                 470                 475                 480

Ser His His His His His His
                485

<210>  53
<211>  494
<212>  PRT
<213>  artificial sequence

<220>
<223>  tandem diabody

<400>  53

Gln Val Gln Leu Val Gln Ser Gly Ala Glu Ala Lys Lys Pro Gly Glu
1               5                   10                  15

Ser Leu Arg Ile Ser Cys Arg Ala Ser Gly Tyr Met Phe Ser Thr Phe
            20                  25                  30

Trp Ile Gly Trp Val Arg Gln Met Pro Gly Lys Gly Leu Glu Trp Val
        35                  40                  45

Ala Ser Ile Tyr Pro Gly Asp Ser Asn Thr Ile Tyr Ser Pro Ser Phe
    50                  55                  60

Gln Gly Gln Val Thr Ile Ser Ala Asp Lys Ser Ile Asn Thr Thr Tyr
65                  70                  75                  80

Leu Gln Trp Ser Gly Leu Lys Ala Ser Asp Thr Ala Thr Tyr Tyr Cys
                85                  90                  95

Ala Lys Ile Arg Asp Gly Tyr Ser Tyr Asp Ala Phe Asp Leu Trp Gly

            100                 105                 110

Gln Gly Thr Met Val Thr Val Ser Ser Gly Gly Ser Gly Gly Ser Gly
        115                 120                 125

Gly Ser Ser Tyr Val Leu Thr Gln Pro Ser Ser Val Ser Val Ala Pro
    130                 135                 140

Gly Gln Thr Ala Thr Ile Ser Cys Gly Gly His Asn Ile Gly Ser Lys
145                 150                 155                 160

Asn Val His Trp Tyr Gln Gln Arg Pro Gly Gln Ser Pro Val Leu Val
                165                 170                 175

Ile Tyr Gln Asp Asn Lys Arg Pro Ser Gly Ile Pro Glu Arg Phe Ser
            180                 185                 190

Gly Ser Asn Ser Gly Asn Thr Ala Thr Leu Thr Ile Ser Gly Thr Gln
        195                 200                 205

Ala Met Asp Glu Ala Asp Tyr Tyr Cys Gln Val Trp Asp Asn Tyr Ser
    210                 215                 220

Val Leu Phe Gly Gly Gly Thr Lys Leu Thr Val Leu Gly Gly Ser Gly
225                 230                 235                 240

Gly Ser Gln Val Gln Leu Val Gln Ser Gly Ala Glu Val Lys Lys Pro
                245                 250                 255

Gly Glu Ser Leu Lys Val Ser Cys Lys Ala Ser Gly Tyr Thr Phe Thr
            260                 265                 270

Ser Tyr Tyr Met His Trp Val Arg Gln Ala Pro Gly Gln Gly Leu Glu
        275                 280                 285

Trp Met Gly Ala Ile Glu Pro Met Tyr Gly Ser Thr Ser Tyr Ala Gln
    290                 295                 300

Lys Phe Gln Gly Arg Val Thr Met Thr Arg Asp Thr Ser Thr Ser Thr
305                 310                 315                 320

Val Tyr Met Glu Leu Ser Ser Leu Arg Ser Glu Asp Thr Ala Val Tyr
                325                 330                 335

Tyr Cys Ala Arg Gly Ser Ala Tyr Tyr Tyr Asp Phe Ala Asp Tyr Trp
            340                 345                 350

Gly Gln Gly Thr Leu Val Thr Val Ser Ser Gly Gly Ser Gly Gly Ser
        355                 360                 365

Gly Gly Ser Gln Ser Ala Leu Thr Gln Pro Ala Ser Val Ser Gly Ser
    370                 375                 380

Pro Gly Gln Ser Ile Thr Ile Ser Cys Thr Gly Thr Ser Ser Asp Val
385                 390                 395                 400

Gly Gly Tyr Asn Tyr Val Ser Trp Tyr Gln Gln His Pro Gly Lys Ala
                405                 410                 415

Pro Lys Leu Met Ile Tyr Asp Val Ser Asn Arg Pro Ser Gly Val Ser
            420                 425                 430

Asn Arg Phe Ser Gly Ser Lys Ser Gly Asn Thr Ala Ser Leu Thr Ile
        435                 440                 445

Ser Gly Leu Gln Ala Glu Asp Glu Ala Asp Tyr Tyr Cys Ser Ser Tyr
    450                 455                 460

Thr Ser Ser Ser Thr Leu Ala Tyr Val Phe Gly Thr Gly Thr Lys Leu
465                 470                 475                 480

Thr Val Leu Ala Ala Ala Gly Ser His His His His His His
                485                 490

<210>  54
<211>  490
<212>  PRT
<213>  artificial sequence

<220>
<223>  tandem diabody

<400>  54

Asp Ile Gln Met Thr Gln Ser Pro Ser Ser Leu Ser Ala Ser Val Gly
1               5                   10                  15

Asp Arg Val Thr Ile Thr Cys Arg Ser Ser Thr Gly Ala Val Thr Thr
            20                  25                  30

Ser Asn Tyr Ala Asn Trp Val Gln Gln Lys Pro Gly Lys Ala Pro Lys
        35                  40                  45

Ala Leu Ile Gly Gly Thr Asn Lys Arg Ala Pro Gly Val Pro Ser Arg
    50                  55                  60

Phe Ser Gly Ser Leu Ile Gly Asp Lys Ala Thr Leu Thr Ile Ser Ser

65                  70                  75                  80

Leu Gln Pro Glu Asp Phe Ala Thr Tyr Tyr Cys Ala Leu Trp Tyr Ser
                85                  90                  95

Asn Leu Trp Val Phe Gly Gln Gly Thr Lys Val Glu Ile Lys Gly Gly
            100                 105                 110

Ser Gly Gly Ser Gln Val Gln Leu Val Glu Ser Gly Gly Gly Val Val
        115                 120                 125

Gln Pro Gly Arg Ser Leu Arg Leu Ser Cys Ala Ala Ser Gly Phe Thr
    130                 135                 140

Phe Ser Ser Tyr Ala Met His Trp Val Arg Gln Ala Pro Gly Lys Gly
145                 150                 155                 160

Leu Glu Trp Val Ala Val Ile Ser Tyr Asp Gly Ser Asn Lys Tyr Tyr
                165                 170                 175

Ala Asp Ser Val Lys Gly Arg Phe Thr Ile Ser Arg Asp Asn Ser Lys
            180                 185                 190

Asn Thr Leu Tyr Leu Gln Met Asn Ser Leu Arg Ala Glu Asp Thr Ala
        195                 200                 205

Val Tyr Tyr Cys Ala Arg Asp His Thr Glu Gly Tyr Tyr Tyr Tyr Gly
    210                 215                 220

Met Asp Val Trp Gly Gln Gly Thr Thr Val Thr Val Ser Ser Gly Gly
225                 230                 235                 240

Ser Gly Ser Tyr Glu Leu Thr Gln Pro Pro Ser Val Ser Val Ser Pro
                245                 250                 255

Gly Gln Thr Ala Ser Ile Thr Cys Ser Gly Asp Lys Leu Gly Asp Lys
            260                 265                 270

Tyr Val Ser Trp Tyr Gln Gln Lys Pro Gly Gln Ser Pro Val Leu Val
        275                 280                 285

Ile Tyr Gln Asp Ser Lys Arg Pro Ser Gly Ile Pro Glu Arg Phe Ser
    290                 295                 300

Gly Ser Asn Ser Gly Asn Thr Ala Thr Leu Thr Ile Ser Gly Thr Gln
305                 310                 315                 320

Ala Met Asp Glu Ala Asp Tyr Tyr Cys Gln Ala Trp Asp Ser Ser Thr
                325                 330                 335

Val Val Phe Gly Gly Gly Thr Lys Val Thr Val Leu Gly Gly Ser Gly
            340                 345                 350

Gly Ser Glu Val Gln Leu Val Glu Ser Gly Gly Gly Leu Val Gln Pro
        355                 360                 365

Gly Gly Ser Leu Arg Leu Ser Cys Ala Ala Ser Gly Phe Thr Phe Ser
    370                 375                 380

Thr Tyr Ala Met Asn Trp Val Arg Gln Ala Pro Gly Lys Gly Leu Glu
385                 390                 395                 400

Trp Val Gly Arg Ile Arg Ser Lys Tyr Asn Asn Tyr Ala Thr Tyr Tyr
                405                 410                 415

Ala Asp Ser Val Lys Asp Arg Phe Thr Ile Ser Arg Asp Asp Ser Lys
            420                 425                 430

Asn Ser Leu Tyr Leu Gln Met Asn Ser Leu Lys Thr Glu Asp Thr Ala
        435                 440                 445

Val Tyr Tyr Cys Ala Arg His Gly Asn Phe Gly Asn Ser Tyr Val Ser
    450                 455                 460

Tyr Phe Ala Tyr Trp Gly Gln Gly Thr Leu Val Thr Val Ser Ser Ala
465                 470                 475                 480

Ala Ala Gly Ser His His His His His His
                485                 490

## Description

### Field of the invention:

The invention relates to antigen binding proteins that specifically bind to a complex of matrix metalloproteinasese 1 (MMP1) and the human leukocyte antigen A2 (HLA-A2), and further to multispecific antigen binding proteins that specifically bind to the MMP1/HLA-A2 complex and an antigen present on an immune effector cell such as, for example T-cell or NK-cell. The invention further includes such multispecific antigen binding proteins for use in immunotherapy, in particular for the treatment of a MMP1⁺ cancer.

### Background:

Bispecific antibodies that recruit and activate mediators of cellular immunity at the site of a tumor are employed in the pharmaceutical industry (Weichel et al., 2015, European Pharmaceutical Review, 20:27-32). While these concepts target cell surface antigens, an alternative approach is to target intracellular tumor antigens that are degraded, processed and displayed by MHC class I molecules as peptide/MHC complexes. Proof-of-Concept for targeting the Wilms tumor 1 oncoprotein (WT1) which is over-expressed in different cancers by an antibody specifically binding to the WT1 peptide/HLA complex has been demonstrated (Dao et al., 2013, Sci. Transl. Med 13,5:176).

The matrix metalloproteinase 1 (MMP1) is responsible for cleavage of collagen-I and other extracellular matrix and cell surface-associated molecules. It is expressed during physiological and pathological tissue remodeling with an important role in development, tissue morphogenesis and wound repair. However, its implication in cancer, rheumatoid arthritis, fibrotic disorders and other diseases has been shown as well and therefore attempts have been undertaken to tackle this target for therapeutic intervention. Knock-down or inhibition of MMP1 is also involved in angiogenesis and cell invasion through extracellular matrix degradation and has been validated as an independent prognostic factor in several cancer settings. Consequently, MMP1 holds promises for a *bona-fide* cancer target.

WO2016/156202 describes that MMP1 exerts a dual effect on tumors. On the one hand MMP1 promotes cancer development by inhibiting apoptosis as well as enhancing migration and invasion of cancer cells; on the other hand MMP1 plays a negative role against cancer development via suppressing metastasis in animal models. High levels of MMP1 were discovered in sera of cancer patients compared with normal control group as well as in multiple tumor tissue specimens, such as gastric cancer, breast cancer, and pancreatic cancer (Zhang et al., 2016 Int. J Oncol). MMP1 was demonstrated to be over-expressed at mRNA level and protein level in CRC tissue compared to normal mucosa. Further, MMP1 expression was correlated with CRC lymph node metastasis, distant metastasis and TNM stage (Tian et al., 2015 J Transl Med. 13:337). MMP1 overexpression is associated with aggressive tumor phenotype and unfavorable clinical outcome in upper urinary tract urothelial carcinomas (UTUC) and urinary bladder urothelial carcinomas (UBUC), suggesting it may serve as a novel prognostic marker and therapeutic target (Li et al., 2016 J Surg Oncol.).

MMP1 expression has been demonstrated in tumor cells as well as in cancer stromal cells. In breast cancer MMP1 is expressed in tumor cells, fibroblast and mononuclear inflammatory cells (Sendon-Lago et al., 2014 Breast Cancer Res. 16:505; Bostrom et al., 2011 BMC Cancer 11:348). In colorectal cancer (CRC) MMP1 is expressed in tumor and stromal cells as well as in tumor cell lines (Kahlert et al., 2014 Br J Cancer 110:441; Shiozawa et al., 2000 Mod Pathol. 13:925). In non-small-cell lung carcinoma (NSCLC) the percentage of tumor cells positive for MMP1 is higher than that of tumor-surrounding fibroblasts (Schutz et al., 2002 Tumor Biol. 23:179). A review on early studies suggests more abundant expression in stroma (lung, head and neck squamous-cell carcinoma, CRC) (Westermarck and Kahari, 1999 FASEB J 13:781).

Examples for MMP1 overexpression in cancer have been reported for breast cancer, where MMP1 expression is associated with tumor progression and poor prognosis (Bostrom et al., 2014 BMC Cancer 11:348); esophageal squamous cell carcinoma, where MMP1 expression is associated with advanced stage and poor prognosis (Tao et al., 2012 Onkologie 35:651); gastric cancer, where MMP1 expression is associated with poor tumor cell differentiation and lymph node metastasis (Cai et al., 2012 Mol Med Rep. 5:1438); colorectal cancer, where MMP1 expression is associated with tumor stage, invasion and metastasis (Shiozawa et al., 2000 Mod Pathol. 13:925) and lung cancer, where MMP1 expression is associated with advanced stage and decreased survival (Li et al., 2010 Lung Cancer 69:341).

Further, MMP1 overexpression is associated with metastasis in human cancer. For example peritoneal and lymph node metastasis in gastric cancer (Inoue et al., 1999 Int J Mol Med. 4:73); depth of invasion, lymph node and hepatic metastasis in colorectal cancer (Shiozawa etal, 2002 In J Cancer 97:432).

In angiogenesis MMP1 facilitates migration and invasion of endothelial cells and vascular smooth muscle cells through extracellular matrix degradation (Chen et al., 2013 Mediators Inflamm. 2013:928315). MMP1 proteolytically activates the thrombin receptor PAR1 on endothelical cells and induces expression of VEGF receptor, thereby promoting angiogenesis (Blackburn and Brinckerhoff, 2008 Am J Pathl. 173:1736; Mazor et al., 2013 J Biol Chem. 288:598). Overexpression of MMP1 promotes endothelial cell recruitment in an *in vitro* glioblastoma model (Pullen et al., 2012 J Neurooncol. 106:461). MMP1 knockdown decreases angiogenesis in a melanoma xenograft model (Blackburn et al., 2007 Cancer Res. 67:10849).

Finally, levels of MMP1, PAR1 and VEGF-C are independent prognostic factors in nasopharyngeal carcinoma, hepatocellular carcinoma, and glioma (Yang et al., 2013 Onco Targets Ther 6:1139; Liao et al., 2012 pathol Oncol Res. 18:397; Xu et al., 2013 Cancer Epidemiol. 37:697).

WO2016/156202 describes peptides of MMP1 displayed by HLA (MHC) class I molecules on the cell surface which are tumor-associated. MMP1-specific tumor-associated peptides have been identified based on extensive expression data-mining (e.g. GTEx Portal) and a unique discovery engine (XPRESIDENT®). Such a tumor-associated peptide is MMP1-003 (SEQ ID NO:45).

### Incorporation by reference

All publications, patents, and patent applications mentioned in this specification are herein incorporated by reference to the same extent as if each individual publication, patent, or patent application is specifically and individually indicated to be incorporated by reference.

### Summary of the invention

It is the object of the present invention to provide a highly specific binding protein that selectively recognizes a MMP1/HLA-A2 complex substantially without exhibiting off-target binding activity and demonstrates a high potency for killing MMP1⁺ tumor cells.

For that purpose, a fully human antibody phage-display library has been employed to identify highly specific single-chain antibodies, which recognize the purified MMP1-003 peptide/HLA-A2 complex in ELISA as well as on peptide-pulsed T2 cells. The candidates were finally subjected to cloning into the fully modular tetravalent tandem diabody (TandAb®) format employing different T-cell or NK-cell engaging domains and scaffold architectures (Example 1).

Excellent target binding and specificity could be confirmed for the tandem diabodies in ELISA and on peptide pulsed T2 cells using the MMP1 peptide (MMP1-003)/HLA-complex. Unrelated peptide/HLA-complexes with a high degree of sequence homology to the MMP1 peptide/HLA-complex served as controls to support the excellent specificity and hence the low risk of off-target binding (Example 2). Finally, all tandem diabodies were characterized in binding and cytotoxicity assays on peptide-pulsed T2 cells and several leads on a large panel of cancer cell lines including different MMP1 or HLA-A2 expression profiles, as well as closely related peptide source proteins. EC₅₀ values in the pM range could be demonstrated in combination with an excellent target-specificity. Although some of the anti-MMP1/HLA-A2 Fv domains demonstrated less affine properties, they showed surprisingly pronounced affinities towards the peptide/HLA complex once formatted into tandem diabodies. The tandem diabodies were very potent. The tandem diabodies were surprisingly highly specific in killing of endogenously MMP1 expressing tumor cell lines, even though the B05 clone bound to additional control peptides on peptide-pulsed T2 cells. This indicates that formatting an anti-MMP1/HLA-A2 Fv domain (e.g. B05) into the tandem diabody format is tuning specificity and affinity.

### Brief description of the drawings

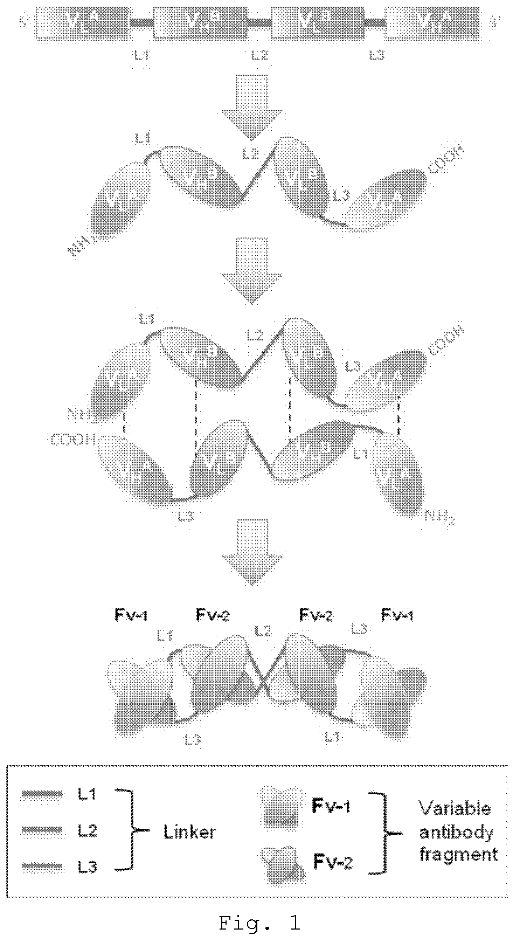

- Figure 1: Schematic representation of gene organization of a multispecific tandem diabody specifically binding to a first antigen A (e.g. MMP1/HLA-A2) and a second antigen B (e.g. CD3 or CD16A). Tandem diabodies are expressed as a single polypeptide comprised of four variable domains connected via short linkers L1, L2 and L3. Following expression, two monomeric polypeptides associate non-covalently head-to-tail to form the functional homodimeric tandem diabody providing four antigen binding sites (Fv-1, Fv-2). L1, L2, L3: Peptide Linker; VH: heavy chain variable domain; VL: light chain variable domain. A, B: antigen specificity.

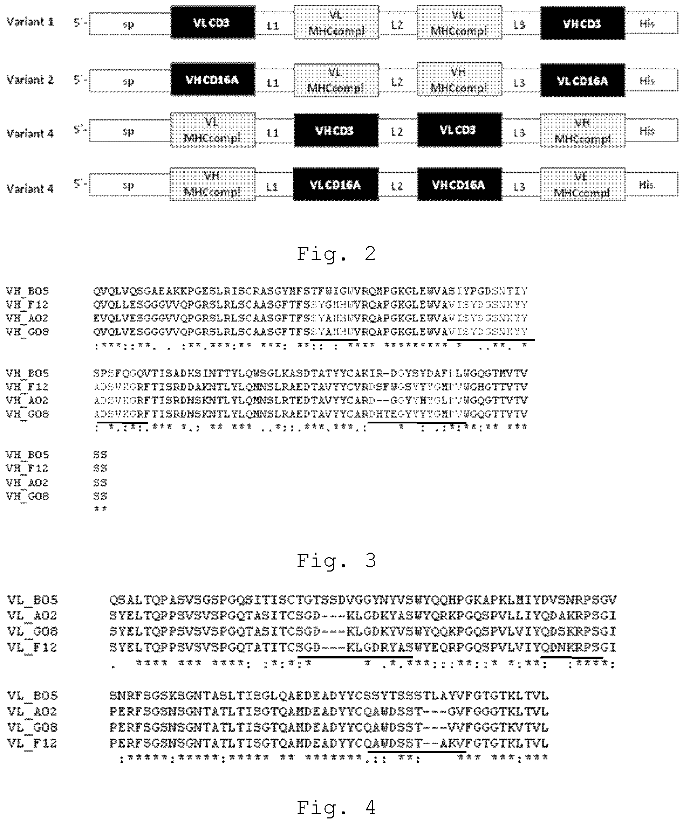

- Figure 2: Schematic representation of a polypeptide and domain order of multispecific tandem diabody specifically binding to MMP1/HLA-A2 and CD3 or CD16A. Tandem diabodies are expressed as a single polypeptide comprised of four variable domains connected via short linkers L1, L2 and L3. First line: variant 1, second line: variant 2, third line: variant 3 and fourth line: variant 4. L1, L2, L3: Linker; VH CD3: heavy chain variable domain binding to CD3; VL CD3: light chain variable domain binding to CD3; "VH MHC complex": heavy chain variable domain binding to MMP1/HLA-A2; and "VL MHC complex": light chain variable domain binding to MMP1/HLA-A2; sp: signal peptide; His: His-Tag.
- Figure 3: Shows an amino acid sequence alignment of the heavy chain variable domains of anti-MMP1/HLA-A2 binding domains of B05, A02, G08 and F12 (SEQ ID NOs:25, 27, 29 and 31). Highly homologous regions between the domains are indicated by a black bar below the alignment.
- Figure 4: shows an amino acid sequence alignment of the light chain variable domains of anti-MMP1/HLA-A2 binding domains of B05, A02, G08 and F12 (SEQ ID NOs:26, 28, 30 and 32). Highly homologous regions between the domains are indicated by a black bar below the alignment.

### Detailed description of the invention

The term "MMP1/HLA-A2" refers to a peptide/MHC complex of a MHC restricted MMP1 peptide bound to an MHC antigen. The binding proteins described herein specifically bind to epitopes of the peptide/MHC complex, i.e. MMP1/HLA-A2 complex. An MMP1 peptide specifically recognized by the binding proteins described herein, when bound to HLA-A2 is MMP1-003 having the amino acid sequence as depicted in SEQ ID NO:45.

The term "binding protein" refers to an immunoglobulin derivative with antigen binding properties; i.e. the binding protein is an antigen binding protein. The binding protein comprises an immunologically functional immunoglobulin portion capable of binding to a target antigen. The immunologically functional immunoglobulin portion may comprise immunoglobulins, or portions thereof, fusionpeptides derived from immunoglobulin portions or conjugates combining immunoglobulin portions that form an antigen binding site. The binding protein comprises at least one antigen binding site which is the region, portion or domain of the binding protein that binds to the target antigen. Each antigen binding site comprises at least the CDRs of the immunoglobulin heavy or light chains from which the antigen binding site was derived. Preferably, the antigen binding site is formed by a variable heavy chain domain (VH) and a variable light chain domain (VL) of an immunoglobulin specifically binding to the same antigen epitope. The variable heavy chain domain comprises three heavy chain complementarity determining regions (CDR): CDR1, CDR2 and CDR3. The variable light chain domain comprises three complementarity determining regions (CDR): CDR1, CDR2 and CDR3. In certain embodiments of the invention the binding protein is devoid of immunoglobulin constant domains. The variable heavy and light chain domains of an antigen binding site may be covalently linked with one another, e.g. by a peptide linker, or non-covalently associate with one another to form an antigen binding site. The binding protein may be a fusion protein comprising at least a heavy and a light chain variable domain specifically binding to the MMP1/HLA-A2 complex. The term "binding protein" refers also to antibody fragments, antibody derivatives or antibody-like binding protein that retain specificity and affinity for their antigen including, for example, IgG-like or non-IgG-like fusionpeptides based on Fv domains either without or with additional consant domains, e.g. Fc-scFv, Fab, Fab', F(ab')₂, Fv fragments, single-chain Fv, tandem single-chain Fv ((scFv)₂), Bi-specific T-cell engagers (BiTE®) or Bi-specific NK-cell engagers (BiKE), dual affinity retargeting antibodies (DART™), diabody, single-chain diabody and tandem diabody (TandAb®); triabody, tribody or Tri-specific NK-cell engagers (TriKE). Dependent on desired features, such as valency, multispecificity, pharmacokinetic and pharmacodynamic properties Fv and/or constant domains and/or additional functional domains may be modularly assembled in different formats or scaffolds, such that, for example, described in Brinkmann and Kontermann, mAbs, 2017, 9(2):182-192 or in Spiess et al., 2015, Molecular Immunology, 67:95-106.

Furthermore, the binding protein may be multivalent, i.e. comprises two or more antigen binding sites. Increasing the valency enhances the functional affinity due to the avidity effect. In some embodiments the binding protein comprises a tag-amino acid sequence for purification.

The present invention relates to a binding protein that specifically binds to a MHC-restricted peptide of matrix metalloproteinase 1 (MMP1) when the peptide is coupled with a major histocompatibility antigen. Hence, the binding protein of the invention specifically recognizes the peptide-MHC complex.

In an embodiment of the invention the binding protein specifically binds to a complex of the peptide MMP1-003 (SEQ ID NO:45) and HLA-A2 (MMP1/HLA-A2).

In a certain embodiment of the invention the binding protein specifically binds to MMP1/HLA-A2, e.g., a complex of the peptide MMP1-003 (SEQ ID NO:45) and HLA-A2, but does substantially not bind to either MMP1 alone or HLA-A2 alone or to a complex of another peptide and HLA-A2, wherein the another peptide is similar, but not identical with MMP1-003, e.g. is at least a 9mer comprising 1, 2, 3, 4 or 5 amino acid residues different from the amino acid sequence depicted in SEQ ID NO:45.

In an embodiment of the invention the binding protein has at least one MMP1/HLA-A2 binding site comprising a light chain variable domain and a heavy chain variable domain. The heavy chain variable domain comprises a heavy chain CDR1, CDR2 and CDR3 and the light chain variable domain comprises a light chain CDR1, CDR2 and CDR3. These heavy chain CDRs may be selected from the group consisting of peptides having an amino acid sequence as depicted in SEQ ID NOs:1, 2, 3, 7, 8, 9, 13, 14, 15, 19, 20 and 21 and these light chain CDRs may be selected from the group of peptides having an amino acid sequence as depicted in SEQ ID NOs:4, 5, 6, 10, 11, 12, 16, 17, 18, 22, 23 and 24.

In a particular embodiment of the invention the MMP1/HLA-A2 binding protein comprises a heavy chain variable domain VH comprising (i) a CDR1 as depicted in SEQ ID NO:1, a CDR2 as depicted in SEQ ID NO:2 and a CDR3 as depicted in SEQ ID NO:3 and a light chain variable domain comprising a CDR1 as depicted in SEQ ID NO:4, a CDR2 as depicted in SEQ ID NO:5 and a CDR3 as depicted in SEQ ID NO:6; or (ii) a heavy chain variable domain VH comprising a CDR1 as depicted in SEQ ID NO:7, a CDR2 as depicted in SEQ ID NO:8 and a CDR3 as depicted in SEQ ID NO:9 and a light chain variable domain comprising a CDR1 as depicted in SEQ ID NO:10, a CDR2 as depicted in SEQ ID NO:11 and a CDR3 as depicted in SEQ ID NO:12; or (iii) a heavy chain variable domain VH comprising a CDR1 as depicted in SEQ ID NO:13, a CDR2 as depicted in SEQ ID NO:14, a CDR3 as depicted in SEQ ID NO:15 and a light chain variable domain comprising a CDR1 as depicted in SEQ ID NO:16, a CDR2 as depicted in SEQ ID NO:17 and a CDR3 as depicted in SEQ ID NO:18; or (iv) a heavy chain variable domain VH comprising a CDR1 as depicted in SEQ ID NO:19, a CDR2 as depicted in SEQ ID NO:20, a CDR3 as depicted in SEQ ID NO:21 and a light chain variable domain comprising a CDR1 as depicted in SEQ ID NO:22, a CDR2 as depicted in SEQ ID NO:23 and a CDR3 as depicted in SEQ ID NO:24

These CDRs may be selected without the surrounding framework sequences of the respective variable domain, which may be further mutated and/or replaced by other suitable framework sequences. The heavy and the light chain variable domains each contribute three CDRs to the MMP1/HLA-A2 binding site of the binding protein. It is known that the six CDRs from a donor framework region can be transferred to an acceptor framework region with retention of antigen binding (Queen et al., PNAS (1988)86:10029-10033, Riechmann et al., Nature (1988)332:323-327). The framework of the heavy chain variable domain may be selected from an immunoglobulin framework of the subtype of gamma (γ); delta (δ), alpha (α), mu (µ), epsilon (ε), a camelid chain or IgNAR (immunoglobulin new antigen receptor); the framework of the light chain variable domain may be selected from an immunoglobulin framework of the subtype of a kappa (κ), lambda (λ), sigma (σ) or Ig-Light-lota. Human heavy and light chain variable domains are preferred.

In a further embodiment of the invention the MMP1/HLA-A2 binding protein comprises a heavy chain variable domain and a light chain variable domain, wherein the heavy chain variable domain is selected from the group consisting of a peptide having an amino acid sequence as depicted in SEQ ID NOs: 25, 27, 29 and 31.

In a further embodiment of the invention the MMP1/HLA-A2 binding protein comprises a heavy chain variable domain and a light chain variable domain wherein the light chain variable domain is selected from the group consisting of a peptide having an amino acid sequence as depicted in SEQ ID NOs: 26, 28, 30 and 32.

In a further embodiment of the invention the MMP1/HLA-A2 binding protein comprises a heavy chain variable domain and a light chain variable domain, wherein the heavy chain variable domain is selected from the group consisting of a peptide having an amino acid sequence as depicted in SEQ ID NOs: 25, 27, 29 and 31 and the light chain variable domain is selected from the group consisting of a peptide having an amino acid sequence as depicted in SEQ ID NOs: 26, 28, 30 and 32.

In a particular embodiment of the invention the MMP1/HLA-A2 binding protein comprises a heavy chain variable domain and a light chain variable domain wherein (i) the heavy chain variable domain has an amino acid sequence as depicted in SEQ ID NO:25 and the light chain variable domain has an amino acid sequence as depicted in SEQ ID NO:26; or (ii) the heavy chain variable domain has an amino acid sequence as depicted in SEQ ID NO:27 and the light chain variable domain has an amino acid sequence as depicted in SEQ ID NO:28; or (iii) the heavy chain variable domain has an amino acid sequence as depicted in SEQ ID NO:29 and the light chain variable domain has an amino acid sequence as depicted in SEQ ID NO:30; or (iv) the heavy chain variable domain has an amino acid sequence as depicted in SEQ ID NO:31 and the light chain variable domain has an amino acid sequence as depicted in SEQ ID NO:32.

In alternative embodiments, the heavy and light chain domains incorporate immunologically active homologues or variants of the CDR or framework sequences described herein. Accordingly in some embodiments, a CDR sequence in a heavy or light chain domain that binds to MMP1/HLA-A2 is similar to, but not identical to, the amino acid sequence depicted in SEQ ID NOs: 1-24. In certain instances, a CDR variant sequence has a sequence identity of 99%, 98%, 97%, 96%, 95%, 94%, 93%, 92%, 91%, 90%, 89%, 88%, 87%, 86%, 85%, 84%, 83%, 82%, 81%, or 80% compared to the sequence of SEQ ID NOs: 1-24 and which is immunologically active.

In further instances, a CDR variant sequence incorporates 1, 2, 3, 4, or 5 conserved amino acid substitutions. Conservative substitutions include amino acid substitutions that substitute a given amino acid with another amino acid of similar characteristics and further include, among the aliphatic amino acids interchange of alanine, valine, leucine, and isoleucine; interchange of the hydroxyl residues serine and threonine, exchange of the acidic residues aspartate and glutamate, substitution between the amide residues asparagine and glutamine, exchange of the basic residues lysine and arginine, and replacements among the aromatic residues phenylalanine and tyrosine.

In yet further instances, a CDR variant sequence incorporates substitutions that enhance properties of the CDR such as increase in stability, resistance to proteases and/or different binding affinities to MMP1/HLA-A2.

In other instances, a CDR variant sequence is modified to change non-critical residues or residues in non-critical regions. Amino acids that are not critical can be identified by known methods, such as affinity maturation, CDR walking mutagenesis, site-directed mutagenesis, crystallization, nuclear magnetic resonance, photoaffinity labeling, or alanine-scanning mutagenesis.

In further alternative embodiments, the MMP1/HLA-A2 binding protein comprises heavy and light chain domains that are immunologically active homologues or variants of heavy and light chain domain sequences provided herein. Accordingly, in some embodiments, a MMP1/HLA-A2 binding protein comprises a heavy or light chain domain sequence that is similar to, but not identical to, the amino acid sequence depicted in SEQ ID NOs: 25-32 or 33-36; 46, 47. In certain instances, a variant heavy or light chain domain sequence has a sequence identity of 99%, 98%, 97%, 96%, 95%, 94%, 93%, 92%, 91%, 90%, 89%, 88%, 87%, 86%, 85%, 84%, 83%, 82%, 81%, or 80% compared to the sequence of SEQ ID NOs: 25-32 or 33-36; 46, 47 and which is immunologically active.

In further instances, a variant heavy or light chain domain sequence incorporates 1, 2, 3, 4, or 5 conserved amino acid substitutions. Conservative substitutions include amino acid substitutions that substitute a given amino acid with another amino acid of similar characteristics and further include, among the aliphatic amino acids interchange of alanine, valine, leucine, and isoleucine; interchange of the hydroxyl residues serine and threonine, exchange of the acidic residues aspartate and glutamate, substitution between the amide residues asparagine and glutamine, exchange of the basic residues lysine and arginine, and replacements among the aromatic residues phenylalanine and tyrosine.

In yet further instances, a variant heavy or light chain domain sequence incorporates substitutions that enhance properties of the CDR such as increase in stability, resistance to proteases and/or binding affinities to MMP1/HLA-A2 or the immune effector target CD3 on T cells or CD16A on NK cells.

In other instances, a variant heavy or light chain domain sequence is modified to change non-critical residues or residues in non-critical regions. Amino acids that are not critical can be identified by known methods, such as affinity maturation, CDR walking mutagenesis, site-directed mutagenesis, crystallization, nuclear magnetic resonance, photoaffinity labeling, or alanine-scanning mutagenesis.

The present invention also provides a MMP1/HLA-A2 binding protein, wherein the MMP1/HLA-A2 antigen binding site is coupled, e.g. conjugated or bound, to at least one further functional moiety.

Thus, the MMP1/HLA-A2 binding protein according to the invention may be multifunctional. The term "multifunctional" as used herein means that a binding protein of the invention exhibits two or more moieties having different biological functions.

In some embodiments the further functional moiety is at least one further functional domain.

In a further embodiment the at least one further functional domain is an effector domain. An "effector domain" comprises a binding site of an antibody specifically binding to an effector cell, e.g. an immune effector cell antigen. The term "effector cell" refers to a cell of the immune system which can stimulate or trigger cytotoxicity, phagocytosis, antigen presentation, cytokine release. Such effector cells are, for example but not limited to, T cells, natural killer (NK) cells, natural killer T (NKT) cells, granulocytes, monocytes, macrophages, dendritic cells, erythrocytes and antigen-presenting cells. Examples of suitable specificities for effector cells include but are not limited to CD2, CD3, CD5, CD28 and other components of the T-cell receptor (TCR) for T-cells; CD16, CD16A, CD38, CD44, CD56, CD69, CD335 (NKp46), CD336 (NKp44), CD337 (NKp30), NKp80, NKG2C and NKG2D (also for T and NKT and γδ T cells) for NK-cells; CD18, CD64 and CD89 for granulocytes; CD18, CD64, CD89 and mannose receptor for monocytes and macrophages; CD64 and mannose receptor for dendritic cells; CD35 for erythrocytes. In certain aspects of the invention those specificities, i.e. cell surface antigens, of effector cells are suitable for mediating cell killing upon binding of a bispecific antibody to such cell surface molecule and, thereby, inducing cytolysis or apoptosis. Preferred immune effector cell antigens according to the invention are CD3 and CD16A.

Thus, the different biological functions may be different specificities for different epitopes, for example different antigens. In this case, the multifunctional MMP1/HLA-A2 binding protein is multispecific, i.e. specifically binds to MMP1/HLA-A2 complex and at least one more antigen, e.g. an antigen displayed on an immune effector cell. Such a multispecific binding protein comprising a first specificity for MMP1/HLA-A2 complex and a second specificity for an immune effector cell antigen is able to cross-link the MMP1/HLA-A2 cell with the immune effector cell displaying the immune effector cell antigen, e.g. CD3 or CD16A and can be used to direct effector cells, e.g. T-cell or NK-cell, to a MMP1⁺/HLA-A2⁺ cell.

Such multispecific binding proteins include, for example, bispecific monoclonal antibodies of the classes IgA, IgD, IgE, IgG or IgM, or antibody fragments, antibody derivatives or antibody-like binding proteins that retain specificity and affinity for their antigen including, for example, IgG-like or non-IgG-like fusionpeptides based on Fv domains either without or with additional consant domains, e.g. Fc-scFv, Fab, Fab', F(ab')₂, Fv fragments, single-chain Fv, tandem single-chain Fv ((scFv)₂), Bi-specific T-cell engagers (BiTE®) or Bi-specific NK-cell engagers (BiKE), dual affinity retargeting antibodies (DART™), diabody, single-chain diabody and tandem diabody (TandAb®); triabody, tribody or Tri-specific NK-cell engagers (TriKE). Dependent on desired features, such as valency, multispecificity, pharmacokinetic and pharmacodynamic properties Fv and/or constant domains and/or additional functional domains may be modularly assembled in different formats or scaffolds, such that, for example, described in Brinkmann and Kontermann, mAbs, 2017, 9(2):182-192 or in Spiess et al., 2015, Molecular Immunology, 67:95-106.

In certain embodiments the multispecific binding protein combines two different antigen specificities and binds with a first binding site to the MMP1/HLA-A2 complex and with a second binding site to either CD3 or CD16A.

CD3 antigen is associated with the T-cell receptor complex on T-cells. In the case where specificity for an effector cell is CD3, the binding of the multispecific binding protein according to the invention to CD3 can trigger the cytotoxic activity of T-cells on target cells. Namely, by bispecific binding of the multispecific binding protein to CD3 and to a target cell, e.g. tumor cell, cell lysis of the target cell may be induced. Multispecific binding proteins with specificity towards CD3 or CD16A and their production have been described (Kipriyanov et al., 1999, Journal of Molecular Biology 293:41-56, Le Gall et al., 2004, Protein Engineering, Design & Selection, 17/4:357-366, Kipriyanov et al., 2009, Methods Mol. Biol. 562:177-193, Weichel et al. 2015, European Pharmaceutical Review 20:27-32). A multispecific binding protein, i.e. tandem diabody that specifically binds to CD3 and CD19⁺ tumor cells has been demonstrated as potent T-cell engaging therapeutic (Reusch et al., 2015, mAbs 7:3, 584-604).

In one particular embodiment the CD3 binding site of the multispecific binding protein specifically binds to human CD3 and, preferably cynomolgus CD3. Examples of such a binding site are disclosed in WO2016/020444. In particular the CD3 binding site may comprise a heavy chain variable domain VH comprising the CDR1, CDR2 and CDR3 of a heavy chain variable domain having the amino acid sequence as depicted in SEQ ID NOs:33 and a light chain variable domain VL comprising the CDR1, CDR2 and CDR3 of the light chain variable domain having the amino acid sequence as depicted in SEQ ID NO:34. In particular, the CD3 binding site may comprise a heavy chain variable domain VH having the amino acid sequence as depicted in SEQ ID NOs:33 and a light chain variable domain VL having the amino acid sequence as depicted in SEQ ID NO:34. These exemplified CD3 binding sites have high affinity to CD3, which is preferred, because the cytotoxicity correlates with the binding to CD3. Alternatively, the CD3 binding site may comprise a heavy chain variable domain VH comprising the CDR1, CDR2 and CDR3 of the heavy chain variable domain and a light chain variable domain VL comprising the CDR1, CDR2 and CDR3 of the light chain variable domain derived from other anti-CD3 domains known in the art, such as, for example, OKT3, UCHT1 or SP34.

The CD16A (FcγRIIIA) antigen is a receptor expressed on the surface of NK-cells. NK-cells possess an inherent cytolytic activity and by bispecific binding of the dimeric antigen-binding molecule according to the invention to CD16A the cytotoxic activity of NK-cell towards the target cell can be triggered. In a particular embodiment of the invention at least one of the heavy chain or light chain variable domains are from an anti-CD16A antibody described in WO 2006/125668, which antibodies specifically recognize the CD16A isoform of human Fc gamma RIII (CD16), but not the CD16B isoform. CD16A exhibits allelic polymorphisms at positions 48 and 158 of the Fc-binding domain. IgG binds with different affinities to CD16A allotypes, due to polymorphisms at position 158. Since affinity to CD16A is implicated as important for the clinical efficacy of therapeutic antibodies, and since the frequencies of these low/high affinity allotypes, 158 V/F, are 0.6 and 0.4, respectively, an anti-CD16A binding site with similar affinity to both allelic forms, and hence potently activating NK-cells in all patients is advantageous. The CD16A specificity with no binding to any CD16B allotype is further advantageous, because broad expression of the non-activating CD16B receptor on granulocytes may act as a sink for non-CD16A specific binding molecules, resulting in decreased efficacy. The anti-CD16A binding site comprising a heavy chain variable domain having the amino acid sequences as depicted in SEQ ID NO:35 and the and a light chain variable domain having the amino acid sequence as depicted in SEQ ID NO:36 recognizes both CD16A allotypes with similar affinity and does not bind to CD16B. The potency of a multispecific binding protein that engages NK cells with the anti-CD16A binding site of SEQ ID NOs:35 and 36for the lysis of CD30+ tumor cells has been demonstrated (Reusch et al., 2014, mAbs 6:3, 727-738). Alternatively, the anti-CD16A antigen binding site may comprise a further affinity maturated Fv domain comprising a heavy chain variable domain having the amino acid sequences as depicted in SEQ ID NO:46 and the and a light chain variable domain having the amino acid sequence as depicted in SEQ ID NO:47.

In preferred embodiments the binding protein is multivalent. The term "multivalent" means that the binding protein provides at least two binding sites, e.g., two, three, four or more binding sites. In particular embodiments, the binding protein provides at least two binding sites for each antigen specificity. For instance, the binding protein may provide at least two binding sites specific for MMP1/HLA-A2 complex and at least two binding sites for an immune effector cell antigen, e.g. CD3 or CD16A. Such a binding protein is at least tetravalent. An example of a tetravalent binding protein is the so-called tandem diabody (TandAb®) further described below. The multivalent, i.e. at least bivalent, binding to each antigen results in a higher avidity, which may cause an increased apparent affinity leading to higher retention on the target or effector cell.

In a further embodiment of the invention the MMP1/HLA-A2 binding protein or multispecific MMP1/HLA-A2 binding protein is a dimer; i.e. comprises two polypeptides with heavy and light chain variable domains for MMP1/HLA-A2 and at least one further antigen.

In an embodiment of the invention a dimeric multispecific binding protein specifically binding to MMP1/HLA-A2 complex and an immune effector cell is provided in the format of a tandem diabody (TandAb®). Such tandem diabodies are constructed by linking four antibody variable binding domains (two heavy-chain variable domains (VH) and two light-chain variable domains (VL) in a single gene construct (Figure 1) enabling homo-dimerization. In such tandem diabodies the linker length is such that it prevents intramolecular pairing of the variable domains so that the molecule cannot fold back upon itself to form a single- chain diabody, but rather is forced to pair with the complementary domains of another chain. The variable domain chains are also arranged such that the corresponding VH and VL domains pair during this dimerization. Following expression from a single gene construct, two identical polypeptide chains fold head-to-tail forming a functional non-covalent homodimer of approximately 105 kDa (Figure 1). Despite the absence of intermolecular covalent bonds, the homodimer is highly stable once formed, remains intact and does not revert back to the monomeric form. Tandem diabodies have a number of properties that provide advantages over traditional monoclonal antibodies and other smaller bispecific molecules. Tandem diabodies contain only antibody variable domains and therefore are contemplated to lack side effects or non-specific interactions that may be associated with an Fc moiety. For example, Fc receptors which can bind to Fc domains are found on numerous cell types such as white blood cells (e.g. basophils, B-cells, eosinophils, natural killer cells, neutrophils and the like) or Kupffer cells. Because tandem diabodies allow for bivalent binding to each of MMP1/HLA-A2 complex and the immune effector cell, the avidity is the same as that of an IgG. The size of a tandem diabody, of approximately 105 kDa, is smaller than that of an IgG, which may allow for enhanced tumor penetration. However, this size is well above the threshold for first-pass renal clearance, offering a pharmacokinetic advantage compared with smaller bispecific formats based on antibody-binding domains or non-antibody scaffolds. Moreover tandem diabodies are advantageous over other bispecific binding proteins such as BiTE® or DART™ molecules based on this pharmacokinetic and avidity properties resulting in longer intrinsic half-lives and rapid cytotoxicity towards target cells. Tandem diabodies are well expressed in host cells, for example, mammalian cells, e.g. CHO cells. It is contemplated that robust upstream and downstream manufacturing processes are available for tandem diabodies.

In one further embodiment the invention provides a tandem diabody binding with a first specificity to a complex of a MHC restricted peptide and a MHC molecule, e.g. HLA-A2 and with a second specificity to an immune effector cell, e.g. through CD3 or CD16A. Such tandem diabody shows surprisingly pronounced affinities towards the peptide/MHC molecule complex due to the tandem diabody format, even if the Fv domains demonstrate less affine properties towards the peptide/MHC molecule complex. Such tandem diabody may be highly specific in killing of target cells, because formatting an antipeptide/MHC molecule complex Fv domain into the tandem diabody format is tuning specificity and affinity.

The bispecific tandem diabodies specifically binding to MMP1/HLA-A2 and an immune effector cell antigen described herein are designed to allow specific targeting of MMP1⁺ tumor cells by engaging an immune effector cell. By engaging surface antigens specifically displayed on immune effector cells, the tandem diabody can crosslink immune effector cells with MMP1⁺ tumor cells in a highly specific fashion, thereby significantly increasing the cytotoxic potential of such immune effector cells. In the context of engaging cytotoxic T-cells via CD3, tandem diabody displays strong, specific and efficient ADCC. Simultaneous binding of this tandem diabody to CD3 and to MMP1/HLA-A2 complex causes crosslinking of target and effector, T-cell activation and mediates the subsequent lysis of the tumor cell.

Therefore, in a further aspect is provided a multispecific, tandem diabody. In some embodiments, a multispecific tandem diabody has specificities to two, three or more different epitopes, wherein two or more epitopes can be of the same antigen target or of different antigen targets. In certain embodiments the multispecific, tandem diabody is bispecific and tetravalent, i.e. comprises four antigen-binding sites. Such a bispecific tandem diabody binds with at least one antigen-binding site, to an immune effector cell and to MMP1/HLA-A2 complex, wherein in certain instances, the tandem diabody binds with two antigen-binding sites to the immune effector cell and with two other antigen-binding sites to MMP1/HLA-A2 complex, i.e. the tandem diabody binds bivalently to each antigen.

In particular embodiments, a bispecific tandem diabody specifically binds to an epitope of an MMP1/HLA-A2 complex. In particular instances the MMP1 is MMP1-003 having the amino acid sequence depicted in SEQ ID NO:45.

In some embodiments, a tandem diabody is specific to MMP1/HLA-A2 complex and CD3, wherein said tandem diabody comprises a first polypeptide and a second polypeptide, each polypeptide having at least four variable chain domains, wherein each polypeptide comprises:
(i) a variable heavy chain (VH) domain specific to MMP1/HLA-A2 complex;
(ii) a variable light chain (VL) domain specific to MMP1/HLA-A2 complex;
(iii) a VH domain specific for CD3, and
(iv) a VL domain specific for CD3.

In particular embodiments the tandem diabodies specific for MMP1/HLA-A2 and CD3 have an affinity to MMP1-003/HLA-A2 complex on MMP1⁺/HLA-A2⁺ cells with a K_{D} of 10 nM or less, 5 nM or less, 1 nM or less, 0.5 nM or less or 0.1 nM or less. The MMP1⁺/HLA-A2⁺ cells can be selected from tumor cells such as, for example, B-CPAP, SW-982 or WM-115 (Example 3).

In a further embodiment a tandem diabody specific for MMP1/HLA-A2 and CD3 binds CD3 and in certain instances, the epsilon chain of CD3 on CD3⁺ cells, particularly T-cells, with a K_{D} of 10 nM or less, 5 nM or less or 2 nM or less.

In some embodiments, each polypeptide of a tandem diabody comprises one of the following combinations of the four variable chain domains: (i) SEQ ID NOs:25; 26, 33 and 34 (ii) SEQ ID NOs:27, 28, 33 and 34, and (iii) SEQ ID NOs:29, 30, 33 and 34.

In other embodiments, a tandem diabody is specific to MMP1/HLA-A2 complex and CD16A, wherein said tandem diabody comprises a first polypeptide and a second polypeptide, each polypeptide having at least four variable chain domains, wherein each polypeptide comprises:
(i) a variable heavy chain (VH) domain specific to MMP1/HLA-A2 complex;
(ii) a variable light chain (VL) domain specific to MMP1/HLA-A2 complex;
(iii) a VH domain specific for CD16A, and
(iv) a VL domain specific for CD16A.

In particular embodiments the tandem diabodies specific for MMP1/HLA-A2 and CD16A have an affinity to MMP1-003/HLA-A2 complex on MMP1⁺/HLA-A2⁺ cells with a K_{D} of 10 nM or less, 5 nM or less, 1 nM or less, 0.5 nM or less or 0.1 nM or less. The MMP1⁺/HLA-A2⁺ cells can be selected from tumor cells such as, for example, B-CPAP, SW-982 or WM-115 (Example 5).

Tandem diabody specific for MMP1/HLA-A2 and CD16A binds CD16A on CD16A⁺ cells, particularly NK-cells, with a K_{D} of 10 nM or less, 5 nM or less or 2 nM or less.

The term "dimer" refers to a complex of two polypeptides. In certain embodiments, the two polypeptides are non-covalently associated with each other, in particular with the proviso that there is no covalent bond between the two polypeptides. In certain instances, the two polypeptides have covalent associations such as disulfide bonds that form to aid in stabilization of the dimer. In certain embodiments, the dimer is homodimeric, i.e. comprises two identical polypeptides. The term "polypeptide" refers to a polymer of amino acid residues linked by amide bonds. The polypeptide is, in certain instances, a single chain fusion protein, which is not branched. In the polypeptide the variable antibody domains are linked one after another. The polypeptide, in other instances, may have contiguous amino acid residues in addition to the variable domain N-terminal and/or C-terminal residues. For example, such contiguous amino acid residues may comprise a Tag sequence, in some instances at the C-terminus, which is contemplated to be useful for the purification and detection of the polypeptide.

In one aspect, each polypeptide of the tandem diabody comprises four variable domains, a variable light chain (VL) and a variable heavy chain (VH) of an immune effector cell binding site as well as a variable light chain (VL) and a variable heavy chain (VH) of a MMP1/HLA-A2 binding site. In certain embodiments, four variable domains are linked by peptide linkers L1, L2 and L3 and in some instances arranged from the N- to the C-terminus as follows (Figure 2): Domain order (the term "IEA" means immune effector cell antigen, e.g., CD3 or CD16A):
(variant 1) VL("IEA")-L1-VH(MMP1/HLA-A2)-L2-VL(MMP1/HLA-A2)-L3-VH("IEA"); or
(variant 2) VH("IEA")-L1-VL(MMP1/HLA-A2)-L2-VH(MMP1/HLA-A2)-L3-VL("IEA"); or
(variant 3) VL(MMP1/HLA-A2)-L1-VH("IEA")-L2-VL("IEA")-L3-VH(MMP1/HLA-A2); or
(variant 4) VH(MMP1/HLA-A2)-L1-VL("IEA")-L2-VH("IEA")-L3-VL(MMP1/HLA-A2).

The length of the linkers influences the flexibility of the tandem diabody according to reported studies. Accordingly, in some embodiments, the length of the peptide linkers L1, L2 and L3 is such that the domains of one polypeptide can associate intermolecularly with the domains of another polypeptide to form the dimeric tandem diabody. In certain embodiments, such linkers are "short", i.e. consist of 0, 1, 2, 3, 4, 5, 6, 7, 8, 9, 10, 11 or 12 amino acid residues. Thus, in certain instances, the linkers consist of about 12 or less amino acid residues. In the case of 0 amino acid residues, the linker is a peptide bond. Such short linkers favor the intermolecular dimerization of two polypeptides by binding and forming correct antigen-binding sites between antibody variable light chain domains and antibody variable heavy chain domains of different polypeptides. Shortening the linker to about 12 or less amino acid residues generally prevents adjacent domains of the same polypeptide chain from intramolecular interaction with each other. In some embodiments, these linkers consist of about 3 to about 10, for example 4, 5 or 6 contiguous amino acid residues.

Regarding the amino acid composition of the linkers, amino acid sequences are selected that do not interfere with the dimerization of the two polypeptides. For example, linkers comprising glycine and serine residues generally provide protease resistance. The amino acid sequence of the linkers can be optimized, for example, by phage-display methods to improve the antigen binding and production yield of the antigen-binding polypeptide dimer. Examples of peptide linkers suitable for a tandem diabody in some embodiments are GGSGGS (SEQ ID NO:42), GGSG (SEQ ID NO:41), GGSGGSGGS (SEQ ID NO:44) or GGSGG (SEQ ID NO:43).

Non-limiting examples of tandem diabodies as described herein are tandem diabodies having an anti-MMP1/HLA-A2 VL and VH domain, an anti-CD3 VL and VH domain, domain order and linker according to Table 1 or an anti-MMP1/HLA-A2 VL and VH domain, an anti-CD16A VL and VH domain according to Table 2.

In another aspect, binding proteins of the invention comprise a further functional moiety which may confer additional effector properties on the binding protein, for example, the functional moiety may be a FcR-binding peptide or the Fc domain of an antibody which, through their capability to bind to Fc receptors, confer effector properties on the binding protein. Alternatively, the functional moiety may be an enzyme that is capable of converting a pro-drug to an active drug. In this way binding proteins of the invention may be used in antibody-dependent enzyme pro-drug therapy (ADEPT). In a further embodiment, the functional moiety is a protein or peptide that confers an increased serum half-life on the binding molecule. An example of such a protein is serum albumin or the Fc portion of IgG, which may increase serum half-life of the binding protein by virtue of its ability to bind to FcRn (the neonatal Fc receptor). In a further embodiment the functional moiety may be a cytokine.

In a further aspect, a binding protein of the invention may be conjugated to a labeling molecule, or a toxin. Where the labeling molecule or toxin is a protein, conjugation to the binding protein may occur through a peptide bond or through chemical conjugation. Thus a binding protein according to this aspect of the invention may be in the form of a fusion protein where the labeling molecule or toxin is linked to the binding protein by a peptide bond, preferably by a peptide linker, or it may be in the form of a chemical conjugate. For the avoidance of doubt, the term conjugation is used herein to mean that two components are physically linked together via a chemical bond, which includes a peptide bond (thus conjugates include fusion proteins), ester linkage, or disulphide bridges.

Conjugation of a binding protein of the invention to a labeling molecule, such as a radiolabel or a fluorescent or luminescent (including chemiluminescent) label allows the binding protein to be used as an immunological staining reagent. Such a reagent may be used in detecting, for example, MMP1/HLA-A2 complexes displayed on cells. Detection of the latter may be particularly useful in the diagnosis of a MMP1⁺ disease or in the monitoring of disease progression or remission.

Where a binding protein of the invention is conjugated to a toxin molecule, such as a ribosyl transferase inhibitor, serine protease inhibitor, guanylyl cylcase activator, calmodulin-dependent adenylate cyclase inhibitor, ribonuclease inhibitor, DNA alkylating agent or mitosis inhibitor (e.g. doxorubicin) and the like, it may be used to target and kill MMP1⁺ cells. Such a binding protein may thus be used as an immunotherapeutic agent.

The MMP1/HLA-A2 binding protein and the multispecific binding protein specifically binding to MMP1/HLA-A2 and an immune effector cell antigen, e.g. CD3 or CD16A (e.g., tandem diabody specifically binding to MMP1/HLA-A2 and CD3 or CD16A) is produced, in some embodiments, by expressing polynucleotides encoding the polypeptide of the binding protein, in particular the polypeptide of the tandem diabody which associates with another identical polypeptide to form the tandem diabody. Therefore, another aspect is a polynucleotide, e.g. DNA or RNA, encoding the polypeptide of a binding protein and tandem diabody as described herein.

The polynucleotide is constructed by known methods such as by combining the genes encoding at least four antibody variable domains either separated by peptide linkers or, in other embodiments, directly linked by a peptide bond, into a single genetic construct operably linked to a suitable promoter, and optionally a suitable transcription terminator, and expressing it in bacteria or other appropriate expression system such as, for example CHO cells. Depending on the vector system and host cell utilized, any number of suitable transcription and translation elements, including constitutive and inducible promoters, may be used. The promoter is selected such that it drives the expression of the polynucleotide in the respective host cell.

In some embodiments, the polynucleotide is inserted into a vector, preferably an expression vector, which represents a further embodiment. This recombinant vector can be constructed according to known methods. A variety of expression vector/host systems may be utilized to contain and express the polynucleotide encoding the polypeptide of the described antigen-binding tandem diabody. Examples of expression vectors for expression in E.coli are pSKK (Le Gall et al, J Immunol Methods. (2004) 285(1): 111-27) or pcDNA5 (Invitrogen) for expression in mammalian cells.

Thus, the binding protein, e.g. tandem diabody, as described herein, in some embodiments, is produced by introducing a vector encoding the polypeptide as described above into a host cell and culturing said host cell under conditions whereby the polypeptide chains are expressed, may be isolated and, optionally, further purified.

In other aspects, provided are pharmaceutical compositions, comprising the MMP1/HLA-A2 binding protein, a tandem diabody, a vector comprising the polynucleotide encoding the polypeptide of the tandem diabody or a host cell transformed or transfected by this vector and at least one pharmaceutically acceptable carrier. The term "pharmaceutically acceptable carrier" includes, but is not limited to, any carrier that does not interfere with the effectiveness of the biological activity of the ingredients and that is not toxic to the patient to whom it is administered. Examples of suitable pharmaceutical carriers are well known in the art and include phosphate buffered saline solutions, water, emulsions, such as oil/water emulsions, various types of wetting agents, sterile solutions etc. Such carriers can be formulated by conventional methods and can be administered to the subject at a suitable dose. Preferably, the compositions are sterile. These compositions may also contain adjuvants such as preservative, emulsifying agents and dispersing agents. Prevention of the action of microorganisms may be ensured by the inclusion of various antibacterial and antifungal agents.

Multispecific binding proteins with high-affinity binding to MMP1/HLA-A2 and an immune effector cell antigen, e.g. CD3 or CD16A are highly active against a large number of MMP1⁺ tumor cells, suggesting that these binding proteins could be active against MMP1⁺ tumors.

The multispecific binding proteins according to the invention, in particular tandem diabodies specifically binding to MMP1/HLA-A2 and CD3 or CD16A, can induce potent cytolysis of MMP1⁺ tumor cells in vitro. The data indicate that high-affinity binding to both MMP1/HLA-A2 and CD3 maximizes tandem diabody-induced T-cell activation or binding to CD16A induces NK cell killing. Therefore, such multispecific binding proteins, e.g. tandem diabodies, are suitable for a therapeutic approach for the treatment of MMP1⁺ tumors such as, for example, breast cancer, esophageal squamous cell carcinoma, gastric cancer, colorectoral cancer, lung cancer, Head and neck squamous cell carcinoma (HNSCC).

Therefore, a further embodiment is a multispecific binding protein, e.g. tandem diabody, specifically binding to MMP1/HLA-A2 complex and either CD3 or CD16A as described herein above for use in the treatment of a MMP1⁺ cancer, e.g., breast cancer, esophageal squamous cell carcinoma, gastric cancer, colorectoral cancer, lung cancer. Such embodiment also includes a method wherein the multispecific binding protein, e.g. tandem diabody, as described herein is administered in an effective dose to a subject, e.g., a patient, for the treatment of a MMP1⁺ cancer (e.g., breast cancer, esophageal squamous cell carcinoma, gastric cancer, colorectoral cancer, lung cancer).

The multispecific binding protein, e.g. tandem diabody, described herein is contemplated for use as a medicament. Administration is effected by different ways, e.g. by intravenous, intraperitoneal, subcutaneous, intramuscular, topical or intradermal injection. In some embodiments, the route of administration depends on the kind of therapy and the kind of compound contained in the pharmaceutical composition. The dosage regimen will be determined by the attending physician and other clinical factors. Dosages for any one patient depends on many factors, including the patient's size, body surface area, age, sex, the particular compound to be administered, time and route of administration, the kind of therapy, general health and other drugs being administered concurrently. An "effective dose" refers to amounts of the active ingredient that are sufficient to affect the course and the severity of the disease, leading to the reduction or remission of such pathology. An "effective dose" useful for treating and/or preventing AML may be determined using known methods.

The following examples should further illustrate the described embodiments without limiting the scope of the invention:

### EXAMPLES

### Example 1: Generation of tandem diabody constructs:

### Cloning of DNA expression constructs encoding tandem diabodies (TandAb^{®})

For expression of bispecific tandem diabodies in CHO cells, coding sequences of all molecules were cloned into a mammalian expression vector system. The anti-HLA-A2^{MMP1-003} scFv domains (SEQ ID NOs:37, 38, 39 and 40) were used to construct anti-HLA-A2^{MMP1-003} /CD3 or anti-HLA-A2^{MMP1-003} /CD16A tandem diabodies in combination with an anti-CD3 heavy and light chain variable domains (SEQ ID NOs:33, 34) or anti-CD16A heavy and light chain variable domains (SEQ ID NOs: 35 and 36; 46 and 47), with domains organized as shown in Table 1 and Figure 2, e.g., the domain orders for variants 2 and 4 of Figure 2 for tandem diabodies with anti-CD16A variable domains. Such a domain organization has been described for a CD30/CD16A tandem diabody molecule (Reusch U et al.; MAbs 2014; 6(3):728-739)). In brief, gene sequences encoding anti-HLA-A2^{MMP1-003} VH and VL domains separated by a peptide linker (VH-linker-VL or VL-linker-VH) were synthesized and subcloned. The resulting construct was digested to generate separate VH and VL coding sequences utilizing a Bam HI restriction site located within the linker sequence. These VH and VL fragments were then ligated with a DNA fragment encoding VH and VL domains of anti-CD3 or anti-CD16A (VH-linker-VL or VL-linker-VH) to yield the final construct. Domain order variants 1 to 4 of anti-HLA-A2^{MMP1-003} /CD3 tandem diabodies are shown in Figure 2. All expression constructs were designed to contain coding sequences for an N-terminal signal peptide and a C-terminal hexahistidine (6xHis)-tag to facilitate antibody secretion and purification, respectively.

### Expression of tandem diabodies in stably transfected CHO cells

A CHO cell expression system (Flp-In^{®}, Life Technologies), a derivative of CHO-K1 Chinese Hamster ovary cells (ATCC, CCL-61) (Kao and Puck, Proc. Natl. Acad Sci USA 1968;60(4):1275-81), was used. Adherent cells were subcultured according to standard cell culture protocols provided by Life Technologies.

For adaption to growth in suspension, cells were detached from tissue culture flasks and placed in serum-free medium. Suspension-adapted cells were cryopreserved in medium with 10% DMSO.

Recombinant CHO cell lines stably expressing secreted tandem diabodies were generated by transfection of suspension-adapted cells. During selection with the antibiotic Hygromycin B or Puromycin dihydrochloride viable cell densities were measured twice a week, and cells were centrifuged and resuspended in fresh selection medium at a maximal density of 0.2x10⁶ viable cells/mL. Cell pools stably expressing tandem diabodies were recovered after 2-3 weeks of selection at which point cells were transferred to standard culture medium in shake flasks. Expression of recombinant secreted proteins was confirmed by performing protein gel electrophoresis. Stable cell pools were cryopreserved in DSMO containing medium.

Tandem diabodies were produced in 10-day fed-batch cultures of stably transfected CHO cells by secretion into the cell culture supernatant. Cell culture supernatants were harvested after 10 days at culture viabilities of typically >75%. Samples were collected from the production cultures every other day and cell density and viability were assessed. On day of harvest, cell culture supernatants were cleared by centrifugation and vacuum filtration before further use.

Protein expression titers and product integrity in cell culture supernatants were analyzed by SDS-PAGE.

### Purification of tandem diabodies

Tandem diabodies were purified from CHO cell culture supernatants in a two-step procedure. The His₆-tagged constructs were subjected to Ni-NTA Superflow chromatography in a first step followed by preparative size exclusion chromatography (SEC) on Superdex 200 in a second step. Eluted tandem diabodies were characterized with regards to their homodimer (tandem diabody) content and pooled if the homodimer content was 90% or higher. Finally, pooled samples were buffer-exchanged and concentrated by ultrafiltration to a typical concentration of >1 mg/mL. Purity and homogeneity (typically >90%) of final samples were assessed by SDS PAGE under reducing and non-reducing conditions, followed by immunoblotting using an anti-His-Tag antibody as well as by analytical SEC, respectively. Purified proteins were stored at aliquots at -80°C until use.

Polypeptides of anti-HLA-A2^{MMP1-003}/CD3 tandem diabodies are shown in Table 1 and Figure 2. Each tandem diabody consists of two identical polypeptides (Figure 1). Both outer linkers L1 and L3 were comprised of six amino acids (SEQ ID NO:42), whereas the central peptide linker L2 varied in length (4-6 amino acids) with the sequences GGSG (SEQ ID NO:41) or GGSGGS (SEQ ID NO:42), respectively.

Anti-HLA-A2^{MMP1-003}/CD16A tandem diabodies consist of two identical polypeptides (Figure 1). Both outer linkers L1 and L3 were comprised of the 9 amino acids GGSGGSGGS (SEQ ID NO:44), whereas the central peptide linker L2 were comprised of 6 amino acids GGSGGS (SEQ ID NO:42), respectively.

Using the anti-HLA-A2^{MMP1-003} variable domains and anti-CD3 or anti-CD16A variable domains a number of tandem diabody molecules was generated that could be stably produced in transfected cell lines and that maintained stability at body temperature as well as after repeated freeze/thaw cycles.

Examples of complete amino acid sequences are shown in Table 1 for the single-chain of anti-HLA-A2^{MMP1-003}/CD3 tandem diabodies TandAbCD3 1 (SEQ ID NO: 48), TandAbCD3 2 (SEQ ID NO: 49) and TandAbCD3 3 (SEQ ID NO:54). The domains within the polypeptide chain are oriented in the following orientation: VL(CD3)-L1-VH(HLA-A2^{MMP1-003}) -L2-VL(HLA-A2^{MMP1-003}) -L3-VH(CD3) (Figure 2).

**Table 1: Polypeptides of anti-HLA-A2^{MMP1-003}/CD3 tandem diabodies**

| Tandem Diabody# | Anti-HLA-A2^{MMP1-003} domain | | Anti-CD3 domain | | Linker | |
|---|---|---|---|---|---|---|
| | VH | VL | VH | VL | L1/L3 | L2 |
| TandAbCD3 1 | SEQ IDNO:25 | SEQ ID NO:26 | SEQ ID NO:33 | SEQ ID NO:34 | SEQ ID NO:42 | SEQ ID NO:41 |
| TandAbCD3 2 | SEQ ID NO:27 | SEQ ID NO:28 | SEQ ID NO:33 | SEQ ID NO:34 | SEQ ID NO:42 | SEQ ID NO:41 |
| TandAbCD3 3 | SEQ ID NO:29 | SEQ ID NO:30 | SEQ ID NO:33 | SEQ ID NO:34 | SEQ ID NO:42 | SEQ ID NO:41 |

Examples of complete amino acid sequences are shown in Table 2 for the single-chain of anti-HLA-A2^{MMP1-003}/CD16A tandem diabodies TandAbCD16A 1 (SEQ ID NO: 50), TandAbCD16A 2 (SEQ ID NO: 51), TandAbCD16A 3 (SEQ ID NO: 52) and TandAbCD16A 4 (SEQ ID NO: 53). The domains within the polypeptide chain are oriented in the following orientation: (i) VH(CD16)-L1-VL(HLA-A2^{MMP1-003}) -L2-VH (HLA-A2^{MMP1-003}) -L3-VL(CD16)(=variant 2) or (ii) VH (HLA-A2^{MMP1-003})-L1-VL (CD16A) -L2-VH (CD16A) -L3-VL (HLA-A2^{MMP1-003}) (=variant 4) (Figure 2).

**Table 2: Polypeptides of anti-HLA-A2^{MMP-003}/CD16A tandem diabodies**

| Tandem Diabody# | Anti-HLA-A2^{MMP1-003} domain | | Anti-CD16A domain | | Linker | |
|---|---|---|---|---|---|---|
| | VH | VL | VH | VL | L1/L3 | L2 |
| TandAbCD16A 1 | SEQ IDNO:27 | SEQ ID NO:28 | SEQ ID NO:46 | SEQ ID NO:47 | SEQ ID NO:44 | SEQ ID NO:42 |
| TandAbCD16A 2 | SEQ ID NO:25 | SEQ ID NO:26 | SEQ ID NO:46 | SEQ ID NO:47 | SEQ ID NO:44 | SEQ ID NO:42 |
| TandAbCD16A 3 | SEQ ID NO:27 | SEQ ID NO:27 | SEQ ID NO:46 | SEQ ID NO:47 | SEQ ID NO:44 | SEQ ID NO:42 |
| TandAbCD16A 4 | SEQ ID NO:25 | SEQ ID NO:26 | SEQ ID NO:46 | SEQ ID NO:47 | SEQ ID NO:44 | SEQ ID NO:42 |

### Example 2: Binding of HLA-A2^{MMP1-003}/CD3 tandem diabodies to effector and target cells

### Methods:

### Culture of cell lines

Human CD3⁺ Jurkat (DSMZ, cat.: ACC 282), cynomolgus CD3⁺ HSC-F (JCRB, cat.: JCRB1164), and human T2 cells (kindly provided by Immatics Biotechnologies GmbH, Tübingen, Germany) were cultured under standard conditions in RPMI 1640 medium supplemented with 2 mM L-glutamine and 100 IU/mL penicillin G sodium and 100 µg/mL streptomycin sulfate (all components from Invitrogen, herein referred to as complete RPMI 1640 medium). All cell lines were cultured at 37°C in a humidified atmosphere with 5% CO₂.

### Isolation of PBMC from buffy coats and enrichment of human T-cells

PBMCs were isolated from buffy coats (German Red Cross, Mannheim, Germany) by density gradient centrifugation. The buffy coat samples were diluted with a two-to-threefold volume of PBS (Invitrogen, cat.: 14190-169), layered on a cushion of Lymphoprep (Stem Cell Technologies, cat.: 07861) and centrifuged at 800 x g for 25 min at room temperature w/o brake. PBMC located in the interface were collected and washed 3 times with PBS before they were cultured in complete RPMI 1640 medium supplemented with 10% human pool serum (Sigma, cat.: H4522) instead 10% FCS overnight without stimulation. For the enrichment of T-cells PBMC were harvested from overnight cultures and used for one round of negative selection using the EasySep™ Human T-Cell Enrichment Kit (Stem Cell Technologies, cat.: 19051) for the immunomagnetic isolation of untouched human T-cells and the Big Easy EasySep™ Magnet (Stem Cell Technologies, cat.: 18001) according to the manufacturer's instructions.

### Peptide-pulsing of T2 cells

T2 cells were resuspended in complete RPMI 1640 medium supplemented with 100 µM of the indicated peptides (Immatics Biotechnologies GmbH, Tübingen, Germany) and incubated for 15 h at 37°C. After incubation, cells were washed and used in binding or cytotoxicity assays.

### Cell binding assays and flow cytometric analysis

Aliquots of the indicated cells were incubated with 100 µL of serial dilutions of His-tagged antibodies (scFv or tandem diabodies) in FACS buffer (PBS, Invitrogen, cat.: 14190-169) containing 2% heat-inactivated FCS (Invitrogen, cat.: 10270-106), 0.1% sodium azide (Roth, Karlsruhe, Germany, cat.: A1430.0100) for 45 min on ice. After repeated washing with FACS buffer, cell-bound antibodies were detected with 10 µg/mL anti-His mAb 13/45/31-2 (Dianova, Hamburg, Germany, cat.: DIA910-1MG) followed by 15 µg/mL FITC-conjugated goat anti-mouse antibody (Dianova, cat.: 115-095-062). The cells were then washed again and resuspended in 0.2 mL of FACS buffer containing 2 µg/mL propidium iodide (PI) (Sigma, cat.: P4170) in order to exclude dead cells. The fluorescence of 2-5 x 10³ living cells was measured using a Beckman-Coulter FC500 MPL flow cytometer using the MXP software (Beckman-Coulter, Krefeld, Germany) or a Millipore Guava EasyCyte flow cytometer (Merck Millipore, Schwalbach, Germany). Mean fluorescence intensities of the cell samples were calculated using CXP software (Beckman-Coulter) or Incyte software (Merck Millipore, Schwalbach, Germany). After subtracting the fluorescence intensity values of the cells stained with the secondary and tertiary reagents alone, the values were used for non-linear regression analysis using the GraphPad Prism software (GraphPad Prism version 6.00 for Windows, GraphPad Software, La Jolla California USA). For the calculation of K_{D}, the equation for one-site-binding (hyperbola) was used.

### Results:

The results of the cell binding assays are summarized in Table 3 for binding to CD3⁺ cells, and in Table 4 for binding to peptide-pulsed T2 cells. Comparative CD19/CD3 tandem diabody and comparative HSA/CD3 tandem diabody were used as a positive control on human CD3⁺ cells. In binding experiments on human CD19⁺ T2 cells CD19/CD3 tandem diabody was used as a positive control and HSA/CD3 tandem diabody was used as a negative control.

All HLA-A2^{MMP1-003}/CD3 tandem diabodies exhibit high affinity binding and sigmoid dose-response curves on CD3⁺ cells. Only TandAbCD3 1 and TandAbCD3 2 tandem diabodies with a cross-species anti-CD3 Fv domain show binding to cynomolgus CD3⁺ cell line HSC-F with similar apparent affinity (1 nM - 3 nM) as to human CD3⁺ cells (1 nM - 4 nM).
TandAbCD3 1 and TandAbCD3 2 HLA-A2^{MMP1-003}/CD3 tandem diabodies exhibit remarkable affinities to MMP1-003 pulsed T2 cells, 3 nM and 40 nM respectively, but no binding to non-pulsed T2 cells.

TandAbCD3 1 tandem diabody with the B05 MMP1 target domain exhibited a >200-fold higher apparent affinity relative to the B05 single chain Fv (scFv), and TandAbCD3 2 tandem diabody with the A02 MMP1 target domain a >2 fold higher apparent affinity than the corresponding scFv A02.

T2 cells were pulsed with 11 different MHC restricted 9mer control peptides which differ from MMP1-003 (SEQ ID NO:45) in four of the nine amino acid residues. On T2 cells that were pulsed with control peptides, HLA-A2^{MMP1-003}/CD3 tandem diabody TandAb 1 showed only high affinity binding to one of the control peptides-pulsed on T2 cells, and no or only low affinity binding (K_{D} values >100 nM) on the T2 cells pulsed with 10 other control peptides. HLA-A2^{MMP1-003}/CD3 tandem diabody TandAbCD3 2 exhibited no or only low affinity binding (K_{D} values >100 nM) on T2 cells pulsed with 11 different control peptides. CD19/CD3 control tandem diabody used as a positive control in each assay showed high affinity binding to pulsed and non-pulsed T2 cells in the range between 3 nM and 11 nM, and HSA/CD3 control tandem diabody showed no substantial binding signals in all binding assays.

Although the scFv's for B05 and A02 showed less affine properties, the affinity towards MMP1/HLA-A2 complex on peptide-pulsed T2 cells was very pronounced after reformatting the B05 and A02 Fv domains into the format of tandem diabodies.

Binding to CD3⁺ cells:

**Table 3: Apparent affinities of anti-HLA-A2^{MMP1-003}/CD3 tandem diabodies and control antibodies in binding assays on CD3⁺ cells.**

| | | | K_{D} values [nM] determined in cell binding assays (on ice), mean K_{D} values of 2 independent experiment are presented | | |
|---|---|---|---|---|---|
| Antibody | Target domain | Effector domain | Human CD3⁺ Jurkat | Human CD3⁺ primary T-cells | Cyno CD3⁺ HSC-F |
| TandAbCD3 1 | B05 | CD3 | 1.1 | 1.7 | 1.3 |
| TandAbCD3 2 | A02 | CD3 | 3.6 | 2.2 | 2.8 |
| Control TandAb CD19/CD3 | Anti-CD19 | CD3 | 0.4 | 0.3 | no |
| Control TandAb HSA/CD3 | Anti-HSA | CD3 | 0.6 | 0.4 | no |
| scFv B05 | B05 | no | no | no | no |
| scFv A02 | A02 | no | no | no | no |

Binding to peptide-pulsed T2 cells:

**Table 4: Apparent affinities (K_{D} values in nM) of anti-HLA-A2^{MMP1-003}/CD3 tandem diabodies in cell binding assays on peptide-pulsed T2 cells. ^{#}, mean of two independent experiments; no, no substantial binding signal; n.a., not applicable.**

| construct | TandAb CD3 1 | TandAb CD3 2 | Control TandAbC D19/CD3 | Control TandAB HSA/CD3 | scFv_ 202.S | scFv_ 203.S |
|---|---|---|---|---|---|---|
| Effector domain | CD3 | CD3 | CD3 | CD3 | n.a. | n.a. |

| Target domain | B05 | A02 | anti-CD19 | anti-HSA | B05 | A02 |
|---|---|---|---|---|---|---|
| No peptide^{#} | no | >500 | 5.1 | no | no | no |
| MMP1-003^{#} | 3.0 | 39.2 | 7.3 | no | 615.9 | 108.4 |

### Example 3: Cytotoxic activity of HLA-A2^{MMP1-003}/CD3 tandem diabodies on peptide-pulsed T2 and tumor cell lines

### Methods:

### Culture of cell lines

B-CPAP (DSMZ, cat.: ACC 273), KMS-27 (JCRB, cat.: JCRB1188), COLO-205 (kindly provided by Dr. G. Moldenhauer, DKFZ, Heidelberg, Germany), JVM-2 (DSMZ, cat.: ACC 12), BXPC-3 (DSMZ, cat.: ACC 760), RPMI-8226 (DSMZ, cat.: ACC 402), MM.1S (ATCC, cat.: CRL-2974), KARPAS-299 (DSMZ, cat.: ACC 31), and T2 cells (kindly provided by Immatics Biotechnologies GmbH, Tübingen, Germany) were cultured under standard conditions in RPMI 1640 medium supplemented with 10% heat-inactivated FCS, 2 mM L-glutamine and 100 IU/mL penicillin G sodium and 100 µg/mL streptomycin sulfate (all components from Invitrogen, herein referred to as complete RPMI 1640 medium). MCF-7 (kindly provided by Dr. G. Moldenhauer, DKFZ, Heidelberg, Germany) were cultured in complete RPMI 1640 medium supplemented with 1 mM pyruvate (all components from Invitrogen). NCI-H929 (DSMZ, cat.: ACC 163) were cultured in RPMI 1640 medium supplemented with 20% heat-inactivated FCS, 2 mM L-glutamine and 100 IU/mL penicillin G sodium and 100 µg/mL streptomycin sulfate, 1 mM pyruvate, and 50 µM β-mercaptoethanol (all components from Invitrogen). SW-982 (ATCC, cat.: HTB-93) and HeLa (kindly provided by Dr. G. Moldenhauer, DKFZ, Heidelberg, Germany) were cultured in DMEM medium supplemented with 10% heat-inactivated FCS, 2 mM L-glutamine and 100 IU/mL penicillin G sodium and 100 µg/mL streptomycin sulfate (all components from Invitrogen). WM-115 (ATCC, cat.: CRL-1675) were cultured in in MEM medium supplemented with 10% heat-inactivated FCS, 2 mM L-glutamine and 100 IU/mL penicillin G sodium and 100 µg/mL streptomycin sulfate (all components from Invitrogen). ES-2 (ATCC, cat.: ARL-1978) were cultured in in McCoy's 5A medium supplemented with 10% heat-inactivated FCS, 2 mM L-glutamine and 100 IU/mL penicillin G sodium and 100 µg/mL streptomycin sulfate (all components from Invitrogen). All cell lines were cultured at 37°C in a humidified atmosphere with 5% CO₂.

### Isolation of PBMC from buffy coats and enrichment of human T-cells

PBMCs were isolated from buffy coats (German Red Cross, Mannheim, Germany) by density gradient centrifugation. The buffy coat sample was diluted with a two-to-threefold volume of PBS (Invitrogen, cat.: 14190-169), layered on a cushion of Lymphoprep (Stem Cell Technologies, cat.: 07861) and centrifuged at 800 x g for 25 min at room temperature w/o brake. PBMC located in the interface were collected and washed 3 times with PBS before they were cultured in complete RPMI 1640 medium supplemented with 10% human pool serum (Sigma, cat.: H4522) instead 10% FCS overnight without stimulation. For the enrichment of T-cells PBMC were harvested from overnight cultures and used for one round of negative selection using the EasySep™ Human T-Cell Enrichment Kit (Stem Cell Technologies, cat.: 19051) for the immunomagnetic isolation of untouched human T-cells and the Big Easy EasySep™ Magnet (Stem Cell Technologies, cat.: 18001) according to the manufacturer's instructions.

### 4 h calcein-release cytotoxicity assays

For calcein-release cytotoxicity assays the indicated target cells were harvested from cultures, washed with RPMI 1640 medium without FCS, and labeled with 10 µM calcein AM (Invitrogen/Molecular Probes, cat.: C3100MP) for 30 min in RPMI medium without FCS at 37°C. After gently washing the labeled cells were resuspended in complete RPMI medium (RPMI 1640 medium supplemented with 10% heat-inactivated FCS, 4 mM L-glutamine, 100 U/mL penicillin G sodium, 100 µg/mL streptomycin sulfate) to a density of 1x10⁵/mL. 1x10⁴ target cells were then seeded together with enriched primary human T-cells at an E:T ratio of 25:1 and the indicated antibodies in individual wells of a round-bottom 96-well micro plate in a total volume of 200 µL/well in duplicates. Spontaneous release, maximal release and killing of targets by effectors in the absence of antibodies were determined in quadruplicate on each plate.

After centrifugation for 2 min at 200 x g the assay was incubated for 4 h at 37°C in a humidified atmosphere with 5% CO₂. 15 min prior to the end of incubation 20 µL of 10% Triton X-100 in RPMI medium were added to wells containing target cells. 20 µL RPMI medium was added to all other wells. 100 µL cell culture supernatant were harvested from each well after an additional centrifugation for 5 min at 500 x g, and the fluorescence of the released calcein was measured at 520 nm using a fluorescence plate reader (Victor 3, Perkin Elmer). On the basis of the measured counts, the specific cell lysis was calculated according to the following formula: [fluorescence (sample) - fluorescence (spontaneous)] / [fluorescence (maximum) - fluorescence (spontaneous)] x 100%. Fluorescence (spontaneous) represents the fluorescent counts from target cells in the absence of effector cells and antibodies and fluorescence (maximum) represents the total cell lysis induced by the addition of Triton X-100. Sigmoidal dose response curves and EC₅₀ values were calculated by non-linear regression/4-parameter logistic fit using the GraphPad Prism software (GraphPad Prism version 6.00 for Windows, GraphPad Software, La Jolla California USA).

### Results:

In 4 h cytotoxicity assays on peptide-pulsed T2 target cells (Table 5) HLA-A2^{MMP1-003}/CD3 tandem diabodies TandAbCD3 1 and TandAbCD3 2 mediated potent and efficacious lysis of MMP1-003 peptide-pulsed cells with EC₅₀ values below 1 pM, and no lysis of non-pulsed T2 target cells. TandAb CD3 1 exhibited only strong cytotoxic cross-reactivity with one of the control peptides-pulsed T2 target cells which is consistent with the relative high affinity binding of TandAbCD3 1 on the same control peptide-pulsed target cells. Low cytotoxic activity of TandAbCD3 1 (EC₅₀ values >470 pM) was observed on T2 cells pulsed with two other control peptides. No substantial cytotoxic activity was observed on the remaining other 8 control peptides.

HLA-A2^{MMP1-003}/CD3 tandem diabody TandAbCD3 2 exhibited intermediate cytotoxic cross-reactivity only on the same control peptide-pulsed T2 cells as TandAbCD3 1, and on an additional control peptide-pulsed T2 target cells with EC₅₀ values in the range between 25 pM and 110 pM and no cytotoxic activity on T2 cells pulsed with the remaining other 8 control peptides.
CD19/CD3 control tandem diabody used as a positive control in each assay mediated efficient lysis of T2 targets irrespective of peptide-loading with EC₅₀ values in the range between 0.4 pM and 1.3 pM, and the negative control HSA/CD3 tandem diabody mediated no lysis at all.

Cytotoxicity of HLA-A2^{MMP1-003}/CD3 tandem diabodies on peptide-pulsed T2 target cells:

**Table 5: Mean EC₅₀ values [pM] for anti-HLA-A2^{MMP1-003}/CD3 tandem diabodies determined in two independent 4 h calcein-release cytotoxicity assays on peptide-pulsed T2 target cells with enriched human T cells as effector cells at an E:T ratio of 25:1. ^{#},mean of 5 independent experiments; no, no substantial target cell lysis. The amino acid sequences of the control peptides are given in SEQ ID NOs:55-64**

| construct | TandAbCD3 1 T714 | TandAbCD3 2 T717 | CD19/CD3 control TandAb | HSA/CD3 control TandAb |
|---|---|---|---|---|
| Effector domain | CD3 | CD3 | CD3 | CD3 |
| Target domain | B05 | A02 | CD19 | HSA |
| No peptide | no | no | 0.7 | no |
| MMP1-003^{#} | 0.7 | 0.9 | 0.9 | no |

In cytotoxicity assays on tumor cell lines (Table 6) HLA-A2⁺/MMP1⁺ target cell lines B-CPAP and SW-982 were efficiently lysed by all T-cell-engaging anti-HLA-A2/MMP1-003 tandem diabodies TandAbCD3 1 and TandAbCD3 2. Lysis of HLA-A2⁺/MMP1⁺ WM-115 was too low for reliable analysis. Positive control CD19/CD3 tandem diabody not induced reproducible and efficacious lysis of WM-115 target cells.

In assays with HLA-A2⁻/MMP1⁺ target cells TandAbCD3 1 was the only tandem diabody that induced no or only minimal lysis of ES-2 and BXPC-3 cell lines. TandAbCD3 3 reproducibly induced lysis of ES-2 and TandAbCD3 2 induced lysis of both HLA-A2⁻/MMP1⁺ cell lines.

In assays with HLA-A2⁻/MMP1⁻ target cells TandAbCD3 1 ***T*** was the only tandem diabody that induced no (four cell lines) or only minimal lysis (one cell line) of all five tested HLA-A2⁻/MMP1⁻ cell lines. TandAbCD3 2 reproducibly induced lysis of all five and TandAbCD3 3 induced lysis of three out of five HLA-A2⁻/MMP1⁻ target cell lines.

In summary, TandAbCD3 1 exhibits the highest selectivity for HLA-A2⁺/MMP1⁺ target cells in 4 h cytotoxicity assay with enriched T-cells as effector cells. TandAbCD3 1 reproducibly induced lysis of two out of three HLA-A2⁺/MMP1⁺ target cell lines, and lysis with very low efficacy of the third cell line (WM-115). In cytotoxicity assays with single-negative or double-negative target cell lines TandAbCD3 1 showed now cytotoxicity activity at all in 7 out of 11 tested cell lines, and low/or not reproducible lysis in assays with four cell lines. In contrast, TandAbCD3 2 reproducibly induced lysis of 9 out of 11 cell lines; and TandAbCD3 3 reproducibly lysed 5 out of 11 cell lines.

The tandem diabodies were very potent. The tandem diabodies were surprisingly highly specific in killing of endogenously expressing tumor cell lines, even though the B05 clone bound to additional control peptides on peptide-pulsed T2 cells (Example 2). This indicates that formatting an anti-MMP1/HLA-A2 Fv domain (e.g. B05) into the tandem diabody format is tuning specificity and affinity.

Cytotoxicity of HLA-A2^{MMP1-003}/CD3 tandem diabodies on tumor target cell lines:

**Table 6: Mean EC₅₀ values [pM] for anti-HLA-A2^{MMP1-003}/CD3 tandem diabodies determined in two independent 4 h calcein-release cytotoxicity assays on tumor target cell lines with enriched human T cells as effector cells at an E:T ratio of 25:1. ^{#}, mean of 5 independent experiments; no, no substantial target cell lysis.**

| | Tandem diabody | | TandAbCD3 1 | TandAbCD3 2 | TandAbCD3 3 |
|---|---|---|---|---|---|
| | Effector binding domain | | CD3 | CD3 | CD3 |
| | Target binding domain | | B05 | A02 | G08 |

| Cell line | HLA-A2 | MMP1 | Mean EC₅₀ [pM] | | |
|---|---|---|---|---|---|
| B-CPAP | + | + | 236.6 | 66.9 | 705.5 |
| SW-982 | + | + | 155.9 | 30.2 | 507.1 |
| WM-115 | + | + | n.a | n.a | no |
| MCF-7 | + | - | n.a | n.a | n.a |
| KMS-27 | + | - | no | 128.5 | n.a |
| COLO-205 | + | - | n.a | n.a | n.a |
| JVM-2 | + | - | no | 74.2 | 14.4 |
| ES-2 | - | + | no/low | 284.6 | no |
| BXPC-3 | - | + | no | 99.9 | 187.2 |
| RPMI-8226 | - | - | no | 159.8 | no |
| MM.1S | - | - | no/low | 308.6 | n.a |
| KARPAS-299 | - | - | no | 131.6 | 286.4 |
| NCI-H929 | - | - | no | 1721.5 | no |
| HeLa | - | - | no | 337.1 | 381.0 |

**Table 7: tumor cell lines used as target cells in cytotoxicity assays**

| Cell line | HLA-A2 | MMP1 | Disease |
|---|---|---|---|
| B-CPAP | + | + | Thyroid carcinoma |
| SW-982 | + | + | Synovial sarcoma |
| WM-115 | + | + | Melanoma |
| MCF-7 | + | - | Breast carcinoma |
| KMS-27 | + | - | Myeloma |
| COLO-205 | + | - | Colon carcinoma |
| JVM-2 | + | - | B-prolymphocytic leukemia |
| ES-2 | - | + | Clear cell carcinoma |
| BXPC-3 | - | + | Pancreas carcinoma |
| RPMI-8226 | - | - | Multiple myeloma |
| MM.1S | - | - | Myeloma |
| KARPAS-299 | - | - | ALCL |
| NCI-H929 | - | - | Multiple myeloma |
| HeLa | - | - | Cervix carcinoma |

### Example 4: Binding of HLA-A2^{MMP1-003}/CD16A tandem diabodies to NK-cells

### Methods:

### Isolation of PBMC from buffy coats and enrichment of human NK-cells

PBMCs were isolated from buffy coats (German Red Cross, Mannheim, Germany) by density gradient centrifugation. The buffy coat samples were diluted with a two-to-threefold volume of PBS (Invitrogen, cat.: 14190-169), layered on a cushion of Lymphoprep (Stem Cell Technologies, cat.: 07861) and centrifuged at 800 x g for 25 min at room temperature w/o brake. PBMC located in the interface were collected and washed 3 times with PBS before they were cultured in complete RPMI 1640 medium supplemented with 10% human pool serum (Sigma, cat.: H4522) instead 10% FCS overnight without stimulation. For the enrichment of NK-cells PBMC were harvested from overnight cultures and used for one round of negative selection using the EasySep™ Human NK-Cell Enrichment Kit (Stem Cell Technologies, cat.: 19055) for the immunomagnetic isolation of untouched human NK-cells and the Big Easy EasySep™ Magnet (Stem Cell Technologies, cat.: 18001) according to the manufacturer's instructions.

### Cell binding assays and flow cytometric analysis

Aliquots of the indicated cells were incubated with 100 µL of serial dilutions of His-tagged tandem diabodies in FACS buffer (PBS, Invitrogen, cat.: 14190-169) containing 2% heat-inactivated FCS (Invitrogen, cat.: 10270-106), 0.1% sodium azide (Roth, Karlsruhe, Germany, cat.: A1430.0100) for 45 min on ice. After repeated washing with FACS buffer, cell-bound antibodies were detected with 10 µg/mL anti-His mAb 13/45/31-2 (Dianova, Hamburg, Germany, cat.: DIA910-1MG) followed by 15 µg/mL FITC-conjugated goat anti-mouse antibody (Dianova, cat.: 115-095-062). The cells were then washed again and resuspended in 0.2 mL of FACS buffer containing 2 µg/mL propidium iodide (PI) (Sigma, cat.: P4170) in order to exclude dead cells. The fluorescence of 2-5 x 10³ living cells was measured using a Beckman-Coulter FC500 MPL flow cytometer using the MXP software (Beckman-Coulter, Krefeld, Germany) or a Millipore Guava EasyCyte flow cytometer (Merck Millipore, Schwalbach, Germany). Mean fluorescence intensities of the cell samples were calculated using CXP software (Beckman-Coulter) or Incyte software (Merck Millipore, Schwalbach, Germany). After subtracting the fluorescence intensity values of the cells stained with the secondary and tertiary reagents alone, the values were used for non-linear regression analysis using the GraphPad Prism software (GraphPad Prism version 6.00 for Windows, GraphPad Software, La Jolla California USA). For the calculation of K_{D}, the equation for one-site-binding (hyperbola) was used.

### Results:

Apparent affinities of HLA-A2^{MMP1-003}/CD16A tandem diabodies and control tandem diabodies for CD16A⁺ cells were determined in two independent experiments using primary human NK-cells. The results summarized in Table 8 demonstrate high affinity binding of HLA-A2^{MMP1-003}/CD16A tandem diabodies to primary human NK-cells in the range of 3 nM to 5 nM. Control tandem diabodies CD19/CD16A TandAb and EGFRvIII/CD16A TandAb were used as a reference and CD19/CD3 TandAb as a negative control.

Binding to CD16⁺ NK-cells:

**Table 8: Apparent affinities of anti-HLA-A2^{MMP1-003}/CD16A tandem diabodies and control tandem diabodies in binding assays on CD16⁺ NK-cells. Mean KD values of two independent experiments are presented. The variants are shown in Figure 2**

| **construct** | **effect or domain** | **target domain** | **variant** | **Mean K_{D} [nM]** |
|---|---|---|---|---|
| TandAbCD16A 1 | CD16A | A02 | 2 | 5.1 |
| TandAbCD16A 2 | CD16A | B05 | 2 | 3.7 |
| TandAbCD16A 3 | CD16A | A02 | 4 | 3.1 |
| TandAbCD16A 4 | CD16A | B05 | 4 | 5.3 |
| CD19/CD16A | CD16A | CD19 | 2 | 1.6 |
| EGFRvIII/CD 16A | CD16A | EGFRvIII [KE1] | 4 | 11.8 |
| CD19/CD3 | CD3 | CD19 | 1 | no binding |

### Example 5: Cytotoxic activity of HLA-A2^{MMP1-003}/CD16A tandem diabodies on peptide-pulsed T2 and tumor cell lines

### Methods:

### Culture of cell lines

B-CPAP (DSMZ, cat.: ACC 273), KMS-27 (JCRB, cat.: JCRB1188), JVM-2 (DSMZ, cat.: ACC 12), BXPC-3 (DSMZ, cat.: ACC 760), MM.1S (ATCC, cat.: CRL-2974), KARPAS-299 (DSMZ, cat.: ACC 31), and T2 cells (kindly provided by Immatics Biotechnologies GmbH, Tübingen, Germany) were cultured under standard conditions in RPMI 1640 medium supplemented with 10% heat-inactivated FCS, 2 mM L-glutamine and 100 IU/mL penicillin G sodium and 100 µg/mL streptomycin sulfate (all components from Invitrogen, herein referred to as complete RPMI 1640 medium). SW-982 (ATCC, cat.: HTB-93) were cultured in DMEM medium supplemented with 10% heat-inactivated FCS, 2 mM L-glutamine and 100 IU/mL penicillin G sodium and 100 µg/mL streptomycin sulfate (all components from Invitrogen). ES-2 (ATCC, cat.: ARL-1978) were cultured in in McCoy's 5A medium supplemented with 10% heat-inactivated FCS, 2 mM L-glutamine and 100 IU/mL penicillin G sodium and 100 µg/mL streptomycin sulfate (all components from Invitrogen). All cell lines were cultured at 37°C in a humidified atmosphere with 5% CO₂.

### Isolation of PBMC from buffy coats and enrichment of human NK-cells

PBMCs were isolated from buffy coats (German Red Cross, Mannheim, Germany) by density gradient centrifugation. The buffy coat samples were diluted with a two-to-threefold volume of PBS (Invitrogen, cat.: 14190-169), layered on a cushion of Lymphoprep (Stem Cell Technologies, cat.: 07861) and centrifuged at 800 x g for 25 min at room temperature w/o brake. PBMC located in the interface were collected and washed 3 times with PBS before they were cultured in complete RPMI 1640 medium supplemented with 10% human pool serum (Sigma, cat.: H4522) instead 10% FCS overnight without stimulation. For the enrichment of NK-cells PBMC were harvested from overnight cultures and used for one round of negative selection using the EasySep™ Human NK-Cell Enrichment Kit (Stem Cell Technologies, cat.: 19055) for the immunomagnetic isolation of untouched human NK-cells and the Big Easy EasySep™ Magnet (Stem Cell Technologies, cat.: 18001) according to the manufacturer's instructions.

### 4 h calcein-release cytotoxicity assays

For calcein-release cytotoxicity assays the indicated target cells were harvested from cultures, washed with RPMI 1640 medium without FCS, and labeled with 10 µM calcein AM (Invitrogen/Molecular Probes, cat.: C3100MP) for 30 min in RPMI medium without FCS at 37°C. After gently washing the labeled cells were resuspended in complete RPMI medium (RPMI 1640 medium supplemented with 10% heat-inactivated FCS, 4 mM L-glutamine, 100 U/mL penicillin G sodium, 100 µg/mL streptomycin sulfate) to a density of 1x10⁵/mL. 1x10⁴ target cells were then seeded together with enriched primary human NK-cells at an E:T ratio of 5:1 and the indicated antibodies in individual wells of a round-bottom 96-well micro plate in a total volume of 200 µL/well in duplicates. Spontaneous release, maximal release and killing of targets by effectors in the absence of antibodies were determined in quadruplicate on each plate.

After centrifugation for 2 min at 200 x g the assay was incubated for 4 h at 37°C in a humidified atmosphere with 5% CO₂. 15 min prior to the end of incubation 20 µL of 10% Triton X-100 in RPMI medium were added to wells containing target cells. 20 µL RPMI medium was added to all other wells. 100 µL cell culture supernatant were harvested from each well after an additional centrifugation for 5 min at 500 x g, and the fluorescence of the released calcein was measured at 520 nm using a fluorescence plate reader (Victor 3, Perkin Elmer). On the basis of the measured counts, the specific cell lysis was calculated according to the following formula: [fluorescence (sample) - fluorescence (spontaneous)] / [fluorescence (maximum) - fluorescence (spontaneous)] x 100%. Fluorescence (spontaneous) represents the fluorescent counts from target cells in the absence of effector cells and antibodies and fluorescence (maximum) represents the total cell lysis induced by the addition of Triton X-100. Sigmoid dose response curves and EC₅₀ values were calculated by non-linear regression/4-parameter logistic fit using the GraphPad Prism software (GraphPad Prism version 6.00 for Windows, GraphPad Software, La Jolla California USA).

### Results:

In 4 h cytotoxicity assays on peptide-pulsed T2 target cells (Table 9) HLA-A2^{MMP1-003}/CD16A tandem diabodies TandAbCD16A 2 and TandAbCD16A 4 containing the anti-HLA-A2^{MMP1-003} Fv domain B05 exhibited the best selectivity for MMP1-003 among the tested peptide-pulsed target cells. In addition to MMP1-003-pulsed target cells TandAbCD16A 2 and TandAbCD16A 4 mediated also lysis of a control peptide-pulsed cells, but not T2 cells pulsed with 7 other control peptides.
CD19/CD16A control tandem diabody used as a positive control in each assay mediated efficient lysis of T2 targets irrespective of peptide-loading with EC₅₀ values in the range between 3.4 pM and 24 pM, and the negative control EGFRvIII/CD16A tandem diabody mediated no lysis at all.

Cytotoxicity of HLA-A2^{MMP1-003}/CD16A tandem diabodies and control tandem diabodies on MMP1-pulsed T2 target cells:

**Table 9: Mean EC₅₀ values [pM] for anti-HLA-A2^{MMP1-003}/CD16A tandem diabodies and control antibodies determined in two independent 4 h calcein-release cytotoxicity assays on peptide-pulsed T2 target cells with enriched human NK-cells as effector cells at an E:T ratio of 5:1. ^{#}, mean of 7 independent experiments; no, no substantial target cell lysis.**

| | TandAb | TandAbCD16A 1 | TandAbCD16A 2 | TandAbCD16A 3 | TandAbCD16A 4 | CD19/CD16A | EGFRvIII/CD16A |
|---|---|---|---|---|---|---|---|
| | effector domain | CD16A | CD16A | CD16A | CD16A | CD16A | CD16A |
| | target domain | A02 | B05 | A02 | B05 | anti-CD19 | anti-EGFRvIII |
| peptide | variant | 2 | 2 | 4 | 4 | 2 | 4 |
| MMP1-003 | ^{#}EC₅₀ [pM] | 14.7 | 20.9 | 5.7 | 8.8 | 12.7 | no |

In cytotoxicity assays on tumor cell lines (Table 10) HLA-A2^{MMP1-003}/CD16A tandem diabodies mediated lysis of HLA-A2⁺/MMP1⁺ target cell lines B-CPAP and SW-982. However, HLA-A2^{MMP1-003}/CD16A tandem diabodies TandAbCD16A 2 and TandAbCD16A 4 were the only tandem diabodies that did not induce lysis of target cell lines lacking expression of either HLA-A2 (ES-2 and BXPC-3) or MMP1 (KMS-27 and JVM-2) or both (MM.1S and KARPAS-299). Control tandem diabodies targeting other tumor antigens (EGFRvIII, CD19, MM, CD30 and EGFR) demonstrated lysis of antigen-positive tumor cells lacking expression of either HLA-A2 or MMP1 or both.

Cytotoxicity of HLA-A2^{MMP1-003}/CD16A tandem diabodies and control tandem diabodies on tumor target cell lines:

**Table 10: Mean EC₅₀ values [pM] for anti-HLA-A2^{MMP1-003}/CD16A tandem diabodies determined in two independent 4 h calcein-release cytotoxicity assays on tumor target cell lines with enriched human NK-cells as effector cells at an E:T ratio of 5:1. n.t., not tested; no, no substantial target cell lysis.**

| | TandAb | TandAb CD16A 1 | TandAb CD16A 2 | TandAb CD16A 3 | TandAb CD16A 4 | EGFRvII I/CD16A | CD19/CD 16A | MM/CD16 A | CD30/CD 16A | EGFR/CD 16A |
|---|---|---|---|---|---|---|---|---|---|---|
| | effector domain | CD16A | CD16A | CD16A | CD16A | CD16A | CD16A | CD16A | CD16A | CD16A |
| | target domain | A02 | B05 | A02 | B05 | anti-EGFRvII I | anti-CD19 | anti-MM antigen | anti-CD30 | anti-EGFR |
| | variant | 2 | 2 | 4 | 4 | 4 | 2 | 4 | 2 | 4 |
| HLA-A2⁺ /MMP1⁺ | B-CPAP | 2612.0 | 1690.0 | 996.0 | 4801.0 | no | n.t. | n.t. | no | 4.2 |
| | SW-982 | 4290.0 | 2861.0 | 1163.0 | 4666.0 | no | n.t. | n.t. | no | 7.3 |
| HLA-A2⁺ /MMP1 | KMS-27 | 927.0 | no | 217.0 | no | no | n.t. | 17.8 | n.t. | n.t. |
| | JVM-2 | 2475.0 | no | 374.0 | no | no | 16.6 | n.t. | n.t. | n.t. |
| HLA-A2 /MMP1⁺ | ES-2 | 3228.0 | no | 1737.0 | no | no | n.t. | n.t. | no | 5.8 |
| | BXPC-3 | 1268.0 | no | 481.0 | no | no | n.t. | n.t. | 16.3 | 3.1 |
| HLA-A2 /MMP1⁻ | MM.1S | 1018.0 | no | 656.0 | no | no | n.t. | 4.1 | n.t. | n.t. |
| | KARPAS-299 | 1217.0 | no | 223.0 | no | no | n.t. | n.t. | 20.7 | n.t. |

**Sequence Summary**

| SEQ.ID. | Sequence |
|---|---|
| 1 | B05 HCDR1 |
| | TFWIG |
| 2 | B05 HCDR2 |
| | SIYPGDSNTIYSPSFQG |
| 3 | B05 HCDR3 |
| | IRDGYSYDAFDL |
| 4 | B05 LCDR1 |
| | TGTSSDVGGYNYVS |
| 5 | B05 LCDR2 |
| | DVSNRPS |
| 6 | B05 LCDR3 |
| | SSYTSSSTLAYV |
| 7 | A02 HCDR1 |
| | SYAM H |
| 8 | A02 HCDR2 |
| | VISYDGSNKYYADSVKG |
| 9 | A02 HCDR3 |
| | DGGYYHYGLDV |
| 10 | A02 LCDR1 |
| | SGDKLGDKYAS |
| 11 | A02 LCDR2 |
| | QDAKRPS |
| 12 | A02 LCDR3 |
| | QAWDSSTGV |
| 13 | G08 HCDR1 |
| | SYAMH |
| 14 | G08 HCDR2 |
| | VISYDGSNKYYADSVKG |
| 15 | G08 HCDR3 |
| | DHTEGYYYYGMDV |
| 16 | G08 LCDR1 |
| | SGDKLGDKYVS |
| 17 | G08 LCDR2 |
| | QDSKRPS |
| 18 | G08 LCDR3 |
| | QAWDSSTVV |
| 19 | F12 HCDR1 |
| | SYGMH |
| 20 | F12 HCDR2 |
| | VISYDGSNKYYADSVKG |
| 21 | F12 HCDR3 |
| | DSFWGSYYYGMDV |
| 22 | F12 LCDR1 |
| | SGDKLGDRYAS |
| 23 | F12 LCDR2 |
| | QDNKRPS |
| 24 | F12 LCDR3 |
| | QAWDSSTAKV |
| 25 | B05 VH |
| | QVQLVQSGAEAKKPGESLRISCRASGYMFSTFWIGWVRQMPGKGLEWVASIYPGDS NTIYSPSFQGQVTISADKSINTTYLQWSGLKASDTATYYCAKIRDGYSYDAFDLWGQGT MVTVSS |
| 26 | B05 VL |
| | QSALTQPASVSGSPGQSITISCTGTSSDVGGYNYVSWYQQHPGKAPKLMIYDVSNRP SGVSNRFSGSKSGNTASLTISGLQAEDEADYYCSSYTSSSTLAYVFGTGTKLTVL |
| 27 | A02 VH |
| | EVQLVESGGGVVQPGRSLRLSCAASGFTFSSYAMHWVRQAPGKGLEWVAVISYDGS NKYYADSVKGRFTISRDNSKNTLYLQMNSLRTEDTAVYYCARDGGYYHYGLDVWGQG TTVTVSS |
| 28 | A02 VL |
| | SYELTQPPSVSVSPGQTASITCSGDKLGDKYASWYQRKPGQSPVLLIYQDAKRPSGIP ERFSGSNSGNTATLTISGTQAMDEADYYCQAWDSSTGVFGGGTKLTVL |
| 29 | G08 VH |
| | QVQLVESGGGVVQPGRSLRLSCAASGFTFSSYAMHWVRQAPGKGLEWVAVISYDGS NKYYADSVKGRFTISRDNSKNTLYLQMNSLRAEDTAVYYCARDHTEGYYYYGMDVWG QGTTVTVSS |
| 30 | G08 VL |
| | SYELTQPPSVSVSPGQTASITCSGDKLGDKYVSWYQQKPGQSPVLVIYQDSKRPSGIP ERFSGSNSGNTATLTISGTQAMDEADYYCQAWDSSTVVFGGGTKVTVL |
| 31 | F12 VH |
| | QVQLLESGGGVVQPGRSLRLSCAASGFTFSSYGMHWVRQAPGKGLEWVAVISYDGS NKYY ADSVKGRFTISRDDAKNTLYLQMNSLRAEDTAVYYCVRDSFWGSYYYGMDVWGHGT TVTVSS |
| 32 | F12 VL |
| | SYELTQPPSVSVSPGQTATITCSGDKLGDRYASWYEQRPGQSPVLVIYQDNKRPSGI PERFSGSNSGNTATLTISGTQAMDEADYYCQAWDSST--AKVFGTGTKLTVL |
| 33 | VH CD3 |
| | EVQLVESGGGLVQPGGSLRLSCAASGFTFSTYAMNWVRQAPGKGLEWVGRIRSKYN NYATYYADSVKDRFTISRDDSKNSLYLQMNSLKTEDTAVYYCARHGNFGNSYVSYFAY WGQGTLVTVSS |
| 34 | VL CD3 |
| | DIQMTQSPSSLSASVGDRVTITCRSSTGAVTTSNYANWVQQKPGKAPKALIGGTNKRA PGVPSRFSGSLIGDKATLTISSLQPEDFATYYCALWYSNLWVFGQGTKVEIK |
| 35 | VH CD16A |
| | QVQLVQSGAEVKKPGESLKVSCKASGYTFTSYYMHWVRQAPGQGLEWMGIINPSGG STSYAQKFQGRVTMTRDTSTSTVYMELSSLRSEDTAVYYCARGSAYYYDFADYWGQG TLVTVSS |
| 36 | VL CD16A |
| | SYVLTQPSSVSVAPGQTATISCGGHNIGSKNVHWYQQRPGQSPVLVIYQDNKRPSGIP ERFSGSNSGNTATLTISGTQAMDEADYYCQVWDNYSVLFGGGTKLTVL |
| 37 | B05 scFv |
| | QVQLVQSGAEAKKPGESLRISCRASGYMFSTFWIGWVRQMPGKGLEWVASIYPGDS NTIYSPSFQGQVTISADKSINTTYLQWSGLKASDTATYYCAKIRDGYSYDAFDLWGQGT MVTVSSGGSGGSGGSGGSGGSGGSQSALTQPASVSGSPGQSITISCTGTSSDVGGY NYVSWYQQHPGKAPKLMIYDVSNRPSGVSNRFSGSKSGNTASLTISGLQAEDEADYY CSSYTSSSTLAYVFGTGTKLTVLAAAGSEQKLISEEDLNSHHHHHH |
| 38 | A02 scFv |
| | EVQLVESGGGVVQPGRSLRLSCAASGFTFSSYAMHWVRQAPGKGLEWVAVISYDGS NKYYADSVKGRFTISRDNSKNTLYLQMNSLRTEDTAVYYCARDGGYYHYGLDVWGQG TTVTVSSGGSGGSGGSGGSGGSGGSSYELTQPPSVSVSPGQTASITCSGDKLGDKY ASWYQRKPGQSPVLLIYQDAKRPSGIPERFSGSNSGNTATLTISGTQAMDEADYYCQA WDSSTGVFGGGTKLTVLAAAGSEQKLISEEDLNSHHHHHH |
| 39 | F12 scFv |
| | QVQLLESGGGVVQPGRSLRLSCAASGFTFSSYGMHWVRQAPGKGLEWVAVISYDGS NKYYADSVKGRFTISRDDAKNTLYLQMNSLRAEDTAVYYCVRDSFWGSYYYGMDVW GHGTTVTVSSLGGSGGSGGSGGSGGSGGSNASYELTQPPSVSVSPGQTATITCSGD KLGDRYASWYEQRPGQSPVLVIYQDNKRPSGIPERFSGSNSGNTATLTISGTQAMDEA DYYCQAWDSSTAKVFGTGTKLTVLAAAGSEQKLISEEDLNSHHHHHH |
| 40 | G08 scFv |
| | QVQLVESGGGVVQPGRSLRLSCAASGFTFSSYAMHWVRQAPGKGLEWVAVISYDGS NKYYADSVKGRFTISRDNSKNTLYLQMNSLRAEDTAVYYCARDHTEGYYYYGMDVWG QGTTVTVSSLGGSGGSGGSGGSGGSGGSNASYELTQPPSVSVSPGQTASITCSGDK LGDKYVSWYQQKPGQSPVLVIYQDSKRPSGIPERFSGSNSGNTATLTISGTQAMDEAD YYCQAWDSSTVVFGGGTKVTVLAAAGSEQKLISEEDLNSHHHHHH |
| 41 | Linker |
| | GGSG |
| 42 | Linker |
| | GGSGGS |
| 43 | Linker |
| | GGSGG |
| 44 | Linker |
| | GGSGGSGGS |
| 45 | MMP1-003 |
| | YTFSGDVQL |
| 46 | VH CD16A |
| | QVQLVQSGAEVKKPGESLKVSCKASGYTFTSYYMHWVRQAPGQGLEWMGAIEPMY GSTSYAQKFQGRVTMTRDTSTSTVYMELSSLRSEDTAVYYCARGSAYYYDFADYWGQ GTLVTVSS |
| 47 | VL CD16A |
| | SYVLTQPSSVSVAPGQTATISCGGHNIGSKNVHWYQQRPGQSPVLVIYQDNKRPSGIP ERFSGSNSGNTATLTISGTQAMDEADYYCQVWDNYSVLFGGGTKLTVL |
| 48 | TandAbCD3 1 |
| | DIQMTQSPSSLSASVGDRVTITCRSSTGAVTTSNYANWVQQKPGKAPKALIGGTNKRA PGVPSRFSGSLIGDKATLTISSLQPEDFATYYCALWYSNLWVFGQGTKVEIKGGSGGS QVQLVQSGAEAKKPGESLRISCRASGYMFSTFWIGWVRQMPGKGLEWVASIYPGDS NTIYSPSFQGQVTISADKSINTTYLQWSGLKASDTATYYCAKIRDGYSYDAFDLWGQGT MVTVSSGGSGQSALTQPASVSGSPGQSITISCTGTSSDVGGYNYVSWYQQHPGKAP KLMIYDVSNRPSGVSNRFSGSKSGNTASLTISGLQAEDEADYYCSSYTSSSTLAYVFG TGTKLTVLGGSGGSEVQLVESGGGLVQPGGSLRLSCAASGFTFSTYAMNWVRQAPG KGLEWVGRIRSKYNNYATYYADSVKDRFTISRDDSKNSLYLQMNSLKTEDTAVYYCAR HGNFGNSYVSYFAYWGQGTLVTVSSAAAGSHHHHHH |
| 49 | TandAbCD3 2 |
| | DIQMTQSPSSLSASVGDRVTITCRSSTGAVTTSNYANWVQQKPGKAPKALIGGTNKRA PGVPSRFSGSLIGDKATLTISSLQPEDFATYYCALWYSNLWVFGQGTKVEIKGGSGGS EVQLVESGGGVVQPGRSLRLSCAASGFTFSSYAMHWVRQAPGKGLEWVAVISYDGS NKYYADSVKGRFTISRDNSKNTLYLQMNSLRTEDTAVYYCARDGGYYHYGLDVWGQG TTVTVSSGGSGSYELTQPPSVSVSPGQTASITCSGDKLGDKYASWYQRKPGQSPVLLI YQDAKRPSGIPERFSGSNSGNTATLTISGTQAMDEADYYCQAWDSSTGVFGGGTKLT VLGGSGGSEVQLVESGGGLVQPGGSLRLSCAASGFTFSTYAMNWVRQAPGKGLEW VGRIRSKYNNYATYYADSVKDRFTISRDDSKNSLYLQMNSLKTEDTAVYYCARHGNFG NSYVSYFAYWGQGTLVTVSSAAAGSHHHHHH |
| 50 | TandAbCD16A 1 |
| | QVQLVQSGAEVKKPGESLKVSCKASGYTFTSYYMHWVRQAPGQGLEWMGAIEPMY GSTSYAQKFQGRVTMTRDTSTSTVYMELSSLRSEDTAVYYCARGSAYYYDFADYWG QGTLVTVSSGGSGGSGGSSYELTQPPSVSVSPGQTASITCSGDKLGDKYASWYQRK PGQSPVLLIYQDAKRPSGIPERFSGSNSGNTATLTISGTQAMDEADYYCQAWDSSTG VFGGGTKLTVLGGSGGSEVQLVESGGGVVQPGRSLRLSCAASGFTFSSYAMHWVR QAPGKGLEWVAVISYDGSNKYYADSVKGRFTISRDNSKNTLYLQMNSLRTEDTAVYY CARDGGYYHYGLDVWGQGTTVTVSSGGSGGSGGSSYVLTQPSSVSVAPGQTATISC GGHNIGSKNVHWYQQRPGQSPVLVIYQDNKRPSGIPERFSGSNSGNTATLTISGTQA MDEADYYCQVWDNYSVLFGGGTKLTVLAAAGSHHHHHH |
| 51 | TandABCD16A 2 |
| | QVQLVQSGAEVKKPGESLKVSCKASGYTFTSYYMHWVRQAPGQGLEWMGAIEPMY GSTSYAQKFQGRVTMTRDTSTSTVYMELSSLRSEDTAVYYCARGSAYYYDFADYWG QGTLVTVSSGGSGGSGGSQSALTQPASVSGSPGQSITISCTGTSSDVGGYNYVSWY QQHPGKAPKLMIYDVSNRPSGVSNRFSGSKSGNTASLTISGLQAEDEADYYCSSYTS SSTLAYVFGTGTKLTVLGGSGGSQVQLVQSGAEAKKPGESLRISCRASGYMFSTFWI GWVRQMPGKGLEWVASIYPGDSNTIYSPSFQGQVTISADKSINTTYLQWSGLKASDT ATYYCAKIRDGYSYDAFDLWGQGTMVTVSSGGSGGSGGSSYVLTQPSSVSVAPGQT ATISCGGHNIGSKNVHWYQQRPGQSPVLVIYQDNKRPSGIPERFSGSNSGNTATLTIS GTQAMDEADYYCQVWDNYSVLFGGGTKLTVLAAAGSHHHHHH |
| 52 | TandAbCD16A 3 |
| | EVQLVESGGGVVQPGRSLRLSCAASGFTFSSYAMHWVRQAPGKGLEWVAVISYDGS NKYYADSVKGRFTISRDNSKNTLYLQMNSLRTEDTAVYYCARDGGYYHYGLDVWGQ GTTVTVSSGGSGGSGGSSYVLTQPSSVSVAPGQTATISCGGHNIGSKNVHWYQQRP GQSPVLVIYQDNKRPSGIPERFSGSNSGNTATLTISGTQAMDEADYYCQVWDNYSVL FGGGTKLTVLGGSGGSQVQLVQSGAEVKKPGESLKVSCKASGYTFTSYYMHWVRQA PGQGLEWMGAIEPMYGSTSYAQKFQGRVTMTRDTSTSTVYMELSSLRSEDTAVYYC ARGSAYYYDFADYWGQGTLVTVSSGGSGGSGGSSYELTQPPSVSVSPGQTASITCS GDKLGDKYASWYQRKPGQSPVLLIYQDAKRPSGIPERFSGSNSGNTATLTISGTQAM DEADYYCQAWDSSTGVFGGGTKLTVLAAAGSHHHHHH |
| 53 | TandAbCD16A 4 |
| | QVQLVQSGAEAKKPGESLRISCRASGYMFSTFWIGWVRQMPGKGLEWVASIYPGDS NTIYSPSFQGQVTISADKSINTTYLQWSGLKASDTATYYCAKIRDGYSYDAFDLWGQG TMVTVSSGGSGGSGGSSYVLTQPSSVSVAPGQTATISCGGHNIGSKNVHWYQQRPG QSPVLVIYQDNKRPSGIPERFSGSNSGNTATLTISGTQAMDEADYYCQVWDNYSVLF GGGTKLTVLGGSGGSQVQLVQSGAEVKKPGESLKVSCKASGYTFTSYYMHWVRQAP GQGLEWMGAIEPMYGSTSYAQKFQGRVTMTRDTSTSTVYMELSSLRSEDTAVYYCA RGSAYYYDFADYWGQGTLVTVSSGGSGGSGGSQSALTQPASVSGSPGQSITISCTG TSSDVGGYNYVSWYQQHPGKAPKLMIYDVSNRPSGVSNRFSGSKSGNTASLTISGLQ AEDEADYYCSSYTSSSTLAYVFGTGTKLTVLAAAGSHHHHHH |
| 54 | TandAbCD3 3 |
| | DIQMTQSPSSLSASVGDRVTITCRSSTGAVTTSNYANWVQQKPGKAPKALIGGTNKRA PGVPSRFSGSLIGDKATLTISSLQPEDFATYYCALWYSNLWVFGQGTKVEIKGGSGGS QVQLVESGGGVVQPGRSLRLSCAASGFTFSSYAMHWVRQAPGKGLEWVAVISYDGS NKYYADSVKGRFTISRDNSKNTLYLQMNSLRAEDTAVYYCARDHTEGYYYYGMDVWG QGTTVTVSSGGSGGSYELTQPPSVSVSPGQTASITCSGDKLGDKYVSWYQQKPGQSP VLVIYQDSKRPSGIPERFSGSNSGNTATLTISGTQAMDEADYYCQAWDSSTVVFGGGT KVTVLGGSGGSEVQLVESGGGLVQPGGSLRLSCAASGFTFSTYAMNWVRQAPGKGL EWVGRIRSKYNNYATYYADSVKDRFTISRDDSKNSLYLQMNSLKTEDTAVYYCARHGN FGNSYVSYFAYWGQGTLVTVSSAAAGSHHHHHH |

## Claims

1. A binding protein comprising an antigen-binding site specific for a MMP1/HLA-A02 complex comprising:
(a) a heavy chain variable region VH comprising a CDR1 as depicted in SEQ ID NO:1, a CDR2 as depicted in SEQ ID NO:2 and a CDR3 as depicted in SEQ ID NO:3 and a light chain variable region comprising a CDR1 as depicted in SEQ ID NO:4, a CDR2 as depicted in SEQ ID NO:5 and a CDR3 as depicted in SEQ ID NO:6;
(b) a heavy chain variable region VH comprising a CDR1 as depicted in SEQ ID NO:7, a CDR2 as depicted in SEQ ID NO: 8 and a CDR3 as depicted in SEQ ID NO:9 and a light chain variable region comprising a CDR1 as depicted in SEQ ID NO:10, a CDR2 as depicted in SEQ ID NO:11 and a CDR3 as depicted in SEQ ID NO:12;
(c) a heavy chain variable region VH comprising a CDR1 as depicted in SEQ ID NO:13, a CDR2 as depicted in SEQ ID NO:14 a CDR3 as depicted in SEQ ID NO:15 and a light chain variable region comprising a CDR1 as depicted in SEQ ID NO:16, a CDR2 as depicted in SEQ ID NO:17 and a CDR3 as depicted in SEQ ID NO:18;
(d) a heavy chain variable region VH comprising a CDR1 as depicted in SEQ ID NO:19, a CDR2 as depicted in SEQ ID NO:20 a CDR3 as depicted in SEQ ID NO:21 and a light chain variable region comprising a CDR1 as depicted in SEQ ID NO:22, a CDR2 as depicted in SEQ ID NO:23 and a CDR3 as depicted in SEQ ID NO:24;
(d) a heavy chain variable region and a light chain variable region, wherein the heavy chain variable region is selected from the group of polypeptides having an amino acid sequence as depicted in SEQ ID NO:25, 27, 29 and 31;or
(e) a heavy chain variable region and a light chain variable region, wherein the light chain variable region is selected from the group of polypeptides having an amino acid sequence as depicted in SEQ ID NO:26, 28, 30 and 32.

2. The binding protein of claim 1, wherein the antigen binding site comprises a pair of heavy and light chain variable domains selected from polypeptides having amino acid sequences of the group consisting of:
(i) SEQ ID NOs:25 and 26;
(ii) SEO ID NOs: 27 and 28;
(iii) SEQ ID NOs:29 and 30; and
(iv) SEQ ID NOs:31 and 32.

3. The binding protein according to claim 1 or 2, wherein MMP1 has the amino acid sequence as depicted in SEQ ID NO:45.

4. A binding protein, wherein the antigen-binding site of any one of claims 1 to 3 is bound to a further functional moiety.

5. The binding protein of claim 3 or 4, wherein the antigen-binding site is bound to a cytotoxic moiety.

6. A multivalent binding protein comprising an antigen-binding site of any one of claims 1 to 3.

7. The multivalent binding protein of claim 6, wherein the binding protein is multispecific and comprises at least one further antigen binding site with an a specificity different from MMP1/HLA-A02 complex.

8. The multivalent binding protein of claim 7, wherein the binding protein comprises at least one antigen-binding site specific for an immune effector cell.

9. The multivalent binding protein of claim 8, wherein the immune effector cell is selected from the group consisting of T-cell, NK-cell, dendritic cell, NKT-cell, γδ T cell and macrophage.

10. The multivalent binding protein of claim 9, wherein the antigen binding molecule comprises at least one antigen binding site specific for CD3 or CD16A.

11. The multivalent binding protein of claims 8 to 10 comprising at least two antigen binding sites for an immune effector cell.

12. The multivalent binding protein of claims 6 to 11, wherein the binding protein is at least tetravalent.

13. The multivalent binding protein of claim 12, wherein the binding protein comprises at least two antigen binding sites specific for an immune effector cell.

14. The multivalent binding protein of claim 13, wherein the binding protein comprises at least two antigen-binding sites specific for MMP1/HLA-A02 complex and at least two antigen binding sites specific for an immune effector cell antigen selected from the group consisting of CD3 and CD16A.

15. A multivalent binding protein of any one of claims 6 to 14 for use as a medicament
